# EUROPEAN PATENT APPLICATION

(11) **EP 4 215 112 A1**
(43) Date of publication of application: **26.07.2023**
(21) Application number: 21869464.4
(22) Date of filing: 17.09.2021
(51) Int. Cl.: A61B 5/11

(54) **PROCESSING DEVICE, PROGRAM, METHOD, AND PROCESSING SYSTEM**

(30) Priority: 18.09.2020 JP 2020157936
(71) Applicant: IMU Corporation, Tokyo 103-0022 (JP)
(72) Inventor: NAGURA, Takeo, Tokyo 103-0022 (JP); SAKIJI, Yasufumi, Tokyo 103-0022 (JP); HARATO, Kengo, Tokyo 103-0022 (JP); IWAMA, Yu, Tokyo 103-0022 (JP); FUKUDA, Atsushi, Tokyo 103-0022 (JP)
(74) Representative: Reeve, Nicholas Edward
(86) International application number: PCT/JP2021/034364
(87) International publication number: WO 2022/059782

(57) **Abstract**

[Problem] Provided are a processing device, program, method, and processing system which can be more simply used by a user when estimating a condition during exercise or assisting estimation.

[Solution] This processing device comprises: an input/output interface configured to receive an acceleration rate detected, in a wired or wireless manner, from a sensor which is attached to or around the knee of a leg of a human and is for detecting at least the acceleration rate of the human during exercise; a memory configured to store computer readable instructions and the received acceleration rate in addition to a predetermined instruction command; and a processor configured to execute the computer readable instructions, by executing the predetermined instruction command stored in the memory, so as to estimating the condition of the knee joint of the human during exercise based on the acceleration rate.

## Description

### BACKGROUND

### Technical Field

The present disclosure relates to a processing device, program, method, and processing system capable of estimating a condition of a human during exercise or assisting the estimation.

### Related Art

Conventionally, a knee joint is the largest joint in the human body, and it has been known that the knee joint plays a major role in a smooth and stable movement of the leg, in particular, during exercise such as walking and turning in direction. However, the knee joint causes various symptoms due to wear and damage of bones, cartilages, ligaments, and the like of the knee joint. For example, it has been known that knee osteoarthritis caused by wear of cartilages of the knee joint or the like causes pain during exercise such as walking.

For example, Patent Literature 1 discloses a motion evaluation device for evaluating a condition of an evaluation target person's exercise, the device configured to perform analysis by using image data obtained by capturing either one of front and rear images of the target person and image data obtained by capturing either one of right and left images thereof, and calculate a parameter related to a body motion.

### Citation List

### Patent Literature

Patent Literature 1: JP 2016-140591 A

### SUMMARY

However, using such a large-scale evaluation device in order to estimate a condition during exercise or assist the estimation, imposes a heavy burden on a user suffering from some symptoms in the knee joint. Thus, various embodiments according to the present disclosure provide a processing device, program, method, and processing system which can be more simply used by a user when estimating a condition during exercise or assisting the estimation.

According to one aspect of the present disclosure, provided is a "processing device comprising: an input/output interface configured to receive an acceleration rate detected, in a wired or wireless manner, from a sensor which is attached to or around a knee of a leg of a human and is for detecting at least the acceleration rate of the human during exercise; a memory configured to store the received acceleration rate in addition to a predetermined instruction command; and a processor configured to perform processing for estimating a condition of a knee joint of the human during exercise on the basis of the acceleration rate by executing the predetermined instruction command stored in the memory".

According to one aspect of the present disclosure, provided is a "processing device comprising: an input/output interface configured to receive an acceleration rate detected, in a wired or wireless manner, from a sensor which is attached to or around a knee of a leg of a human and is for detecting at least the acceleration rate of the human during exercise; a memory configured to store the received acceleration rate in addition to a predetermined instruction command; and a processor configured to perform processing for outputting an acceleration rate after landing of the leg among the acceleration rates received from the sensor, by executing the predetermined instruction command stored in the memory".

According to one aspect of the present disclosure, provided is a "processing device comprising: an input/output interface configured to receive an acceleration rate detected, in a wired or wireless manner, from a sensor which is attached to or around a knee of a leg of a human and is for detecting at least the acceleration rate of the human during exercise; a memory configured to store the received acceleration rate in addition to a predetermined instruction command; and a processor configured to perform processing for estimating a degree or a prognosis of knee osteoarthritis on the basis of the acceleration rate received from the sensor by executing the predetermined instruction command stored in the memory".

According to one aspect of the present disclosure, provided is a "processing device comprising: an input/output interface configured to receive an acceleration rate detected, in a wired or wireless manner, from a sensor which is attached to or around a knee of a leg of a human and is for detecting at least the acceleration rate of the human during exercise; a memory configured to store the received acceleration rate in addition to a predetermined instruction command; and a processor configured to perform processing for outputting information indicating a condition of a knee joint of the human during exercise estimated on the basis of the acceleration rate by executing the predetermined instruction command stored in the memory".

According to one aspect of the present disclosure, provided is a "program causing a computer comprising an input/output interface configured to receive an acceleration rate detected, in a wired or wireless manner, from a sensor which is attached to or around a knee of a leg of a human and is for detecting at least the acceleration rate of the human during exercise, and a memory configured to store the received acceleration rate, to function as a processor configured to perform processing for estimating a condition of a knee joint of the human during exercise on the basis of the acceleration rate".

According to one aspect of the present disclosure, provided is a "program causing a computer comprising an input/output interface configured to receive an acceleration rate detected, in a wired or wireless manner, from a sensor which is attached to or around a knee of a leg of a human and is for detecting at least the acceleration rate of the human during exercise, and a memory configured to store the received acceleration rate, to function as a processor configured to perform processing for outputting an acceleration rate after landing of the leg among the acceleration rates received from the sensor".

According to one aspect of the present disclosure, provided is a "program causing a computer comprising an input/output interface configured to receive an acceleration rate detected, in a wired or wireless manner, from a sensor which is attached to or around a knee of a leg of a human and is for detecting at least the acceleration rate of the human during exercise, and a memory configured to store the received acceleration rate, to function as a processor configured to perform processing for estimating a degree or a prognosis of knee osteoarthritis on the basis of the acceleration rate received from the sensor".

According to one aspect of the present disclosure, provided is a "program causing a computer comprising an input/output interface configured to receive an acceleration rate detected, in a wired or wireless manner, from a sensor which is attached to or around a knee of a leg of a human and is for detecting at least the acceleration rate of the human during exercise, and a memory configured to store the received acceleration rate, to function as a processor configured to perform processing for outputting information indicating a condition of a knee joint of the human during exercise estimated on the basis of the acceleration rate".

According to one aspect of the present disclosure, provided is a "method to be performed by a processor, in a computer comprising an input/output interface configured to receive an acceleration rate detected, in a wired or wireless manner, from a sensor which is attached to or around a knee of a leg of a human and is for detecting at least the acceleration rate of the human during exercise, and a memory configured to store the received acceleration rate in addition to a predetermined instruction command, the processor executing the predetermined instruction command, the method comprising a step for estimating a condition of a knee joint of the human during exercise on the basis of the acceleration rate".

According to one aspect of the present disclosure, provided is a "method to be performed by a processor, in a computer comprising an input/output interface configured to receive an acceleration rate detected, in a wired or wireless manner, from a sensor which is attached to or around a knee of a leg of a human and is for detecting at least the acceleration rate of the human during exercise, and a memory configured to store the received acceleration rate in addition to a predetermined instruction command, the processor executing the predetermined instruction command, the method comprising a step for outputting an acceleration rate after landing of the leg among the acceleration rates received from the sensor".

According to one aspect of the present disclosure, provided is a "method to be performed by a processor, in a computer comprising an input/output interface configured to receive an acceleration rate detected, in a wired or wireless manner, from a sensor which is attached to or around a knee of a leg of a human and is for detecting at least the acceleration rate of the human during exercise, and a memory configured to store the received acceleration rate in addition to a predetermined instruction command, the processor executing the predetermined instruction command, the method comprising a step for estimating a degree or a prognosis of knee osteoarthritis on the basis of the acceleration rate received from the sensor".

According to one aspect of the present disclosure, provided is a "method to be performed by a processor, in a computer comprising an input/output interface configured to receive an acceleration rate detected, in a wired or wireless manner, from a sensor which is attached to or around a knee of a leg of a human and is for detecting at least the acceleration rate of the human during exercise, and a memory configured to store the received acceleration rate in addition to a predetermined instruction command, the processor executing the predetermined instruction command, the method comprising a step for outputting information indicating a condition of a knee joint of the human during exercise estimated on the basis of the acceleration rate".

According to one aspect of the present disclosure, provided is a "processing system comprising: the processing device described above; and a detection device including an acceleration sensor which is attached to or around a knee of a leg of a human and is for detecting at least the acceleration rate of the human during exercise".

According to the present disclosure, it is possible to provide a processing device, program, method, and processing system which can be more simply used by a user when estimating a condition during exercise or assisting the estimation.

Note that the above effects are merely exemplary for convenience of explanation, and are not restrictive. In addition to or instead of the above effects, it is possible to exhibit any effect described in the present disclosure or an effect obvious to a person skilled in the art.

### BRIEF DESCRIPTION OF THE DRAWINGS

Fig. 1 illustrates a usage state of a processing system 1 according to the present disclosure.
Fig. 2 is a schematic diagram of the processing system 1 according to an embodiment of the present disclosure.
Fig. 3 is a block diagram illustrating a configuration of the processing system 1 according to the embodiment of the present disclosure.
Fig. 4 illustrates an external appearance of a detection device 200 according to the embodiment of the present disclosure.
Fig. 5 illustrates an external appearance of an auxiliary tool 400 according to the embodiment of the present disclosure.
Fig. 6 illustrates one example of an output value detected by the detection device 200 according to the embodiment of the present disclosure.
Fig. 7A illustrates one example of an acceleration rate table stored in a processing device 100 according to the embodiment of the present disclosure.
Fig. 7B illustrates one example of a user table stored in the processing device 100 according to the embodiment of the present disclosure.
Fig. 7C illustrates one example of a KAM conversion table stored in the processing device 100 according to the embodiment of the present disclosure.
Fig. 7D illustrates one example of a prognosis conversion table stored in the processing device 100 according to the embodiment of the present disclosure.
Fig. 7E illustrates one example of an institution table stored in the processing device 100 according to the embodiment of the present disclosure.
Fig. 7F illustrates one example of an auxiliary information table stored in the processing device 100 according to the embodiment of the present disclosure.
Fig. 8 illustrates a processing flow executed in the processing device 100 according to the embodiment of the present disclosure.
Fig. 9 illustrates a processing flow executed in the processing device 100 according to the embodiment of the present disclosure.
Fig. 10 illustrates a processing flow executed in the processing device 100 according to the embodiment of the present disclosure.
Fig. 11 illustrates a processing flow executed in the processing device 100 according to the embodiment of the present disclosure.
Fig. 12 illustrates one example of an acceleration rate detected by the detection device 200 according to the embodiment of the present disclosure.
Fig. 13 illustrates a processing flow executed in the processing device 100 according to the embodiment of the present disclosure.
Fig. 14 illustrates one example of a screen displayed in the processing device 100 according to the embodiment of the present disclosure.
Fig. 15 illustrates one example of a screen displayed in the processing device 100 according to the embodiment of the present disclosure.
Fig. 16 illustrates one example of a screen displayed in the processing device 100 according to the embodiment of the present disclosure.
Fig. 17 illustrates a processing flow related to the generation of a trained estimation model according to the embodiment of the present disclosure.
Fig. 18 illustrates a processing flow executed in the processing device 100 according to the embodiment of the present disclosure.
Fig. 19 illustrates a correlation between an acceleration rate which has been actually measured and a KAM value.
Fig. 20 illustrates a correlation between an output value which has been actually measured and a KAM value.
Fig. 21 illustrates a correlation between an output value which has been actually measured and a KAM value.

### DESCRIPTION OF EXEMPLARY EMBODIMENTS

Various embodiments of the present disclosure will be explained with reference to the accompanying drawings. Note that common components in the drawings are denoted by the same reference numeral.

### 1. Outline of Processing System 1

A processing system 1 according to the present disclosure includes a processing device 100 and a detection device 200, and is used to estimate a condition of a knee of a user (human) or assist the estimation by processing, in the processing device 100, an output value detected by the detection device 200 attached to the user. In particular, the processing system 1 is attached to or around a knee of the user and is used to estimate a degree or a prognosis of knee osteoarthritis, or assist the estimation by using an output value detected by the detection device 200 during the user's exercise.
Therefore, a case where the processing system 1 according to the present disclosure is used to estimate a degree or a prognosis of knee osteoarthritis, or assist the estimation is mainly explained hereinafter. However, the degree or the prognosis of knee osteoarthritis is one example of the condition, and besides, the attachment position of the detection device is merely one example.

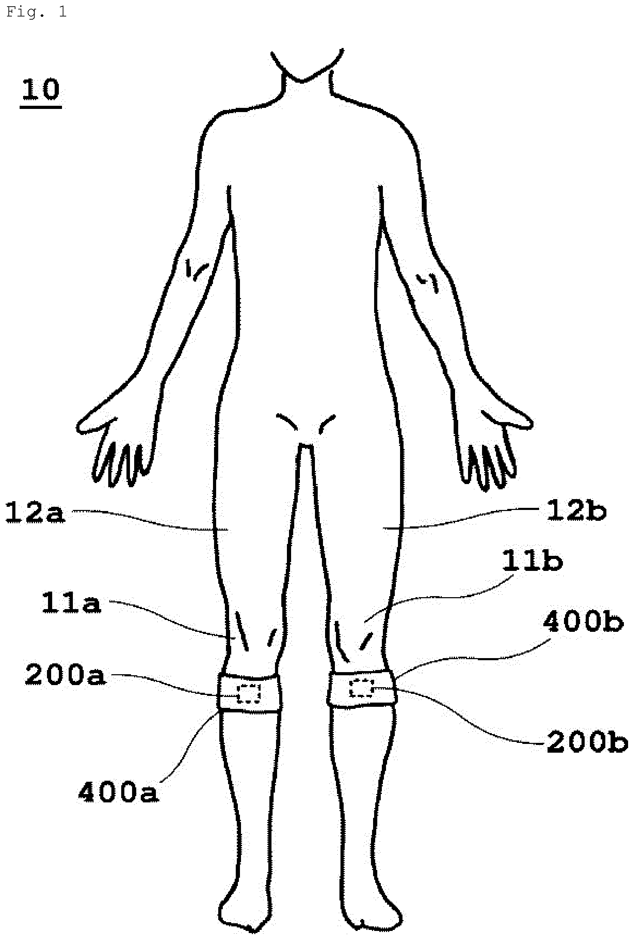

Fig. 1 illustrates a usage state of the processing system 1 according to the present disclosure. Specifically, the diagram illustrates a state in which the detection device 200 of the processing system 1 is being used while being attached to a user 10. According to Fig. 1, a detection device 200a of the processing system 1 is attached to a lower portion of a right knee 11a of a right leg 12a of the user 10 by winding an auxiliary tool 400a around the right leg 12a. Similarly, a detection device 200b of the processing system 1 is attached to a lower portion of a left knee 11b of a left leg 12b of the user 10 by winding an auxiliary tool 400b around the left leg 12b. Thereafter, the user having the detection device 200a and the detection device 200b mounted thereto, is caused to perform a walking exercise in a predetermined direction, and the detection device 200a and the detection device 200b each detect an output value (for example, an acceleration rate) generated during this walking exercise. Then, the detection device 200a and the detection device 200b each transmit the detected output value to the processing device 100 (not illustrated in Fig. 1.) of the processing system 1.

Note that, in the present disclosure, the human to which the detection device 200 is attached may include any person such as a patient, a subject, and a person to be diagnosed. The detection device 200 according to the present disclosure is not limited to a case of being used in a medical institution, for example, and may be used at any place such as an athletic gym, an orthopedic clinic, an osteopathic clinic, or a workplace or a home of the user, for example. Therefore, an attribute of a person having the detection device 200 mounted thereto is irrelevant. In addition, in the present disclosure, an operator merely means a person who operates the processing device 100. Therefore, the operator may be the same person as the user described above, or may be a person different from the user, for example, a medical worker or a gym trainer.

In addition, although Fig. 1 exemplifies a case where the detection device 200a or 200b is mounted below a knee (front side), the detection device may be mounted above the knee, to the knee, or any other part of the leg. Besides, the position where the detection device 200a or 200b is mounted, is not limited to a front surface side of the leg 12a or 12b, but may be either a rear surface side or a side surface side. Furthermore, although Fig. 1 illustrates an example in which two detection devices 200, that is, the detection devices 200a and 200b, are used, the number of detection devices may be obviously one on one foot side, or two or more detection devices may be mounted to a plurality of positions in the legs 12a and/or 12b.

An acceleration sensor is typically used as the detection device 200, and detects an acceleration rate during exercise. However, not only the acceleration sensor but also any sensor capable of detecting the exercise of the user 10, in particular, movements including bending and stretching of the knee, such as a gyro sensor, a geomagnetic sensor, and an expansion/contraction sensor can be used, and it is also possible to use an output value corresponding thereto for estimating the condition of the exercise or assisting the estimation. In addition, a plurality of sensors such as an acceleration sensor and a gyro sensor can be used in combination.

The condition to be estimated or to be assisted in the estimation by the processing system 1 is a condition during exercise. This exercise is typically the user's walking, but may include various other exercises such as running, bending and stretching, and jumping. It is not necessary to perform these exercises only for estimating the condition or assisting the estimation thereof, and for example, the detection device 200 may be mounted to the user 10 on a daily basis so as to detect an output value during exercise in daily life.

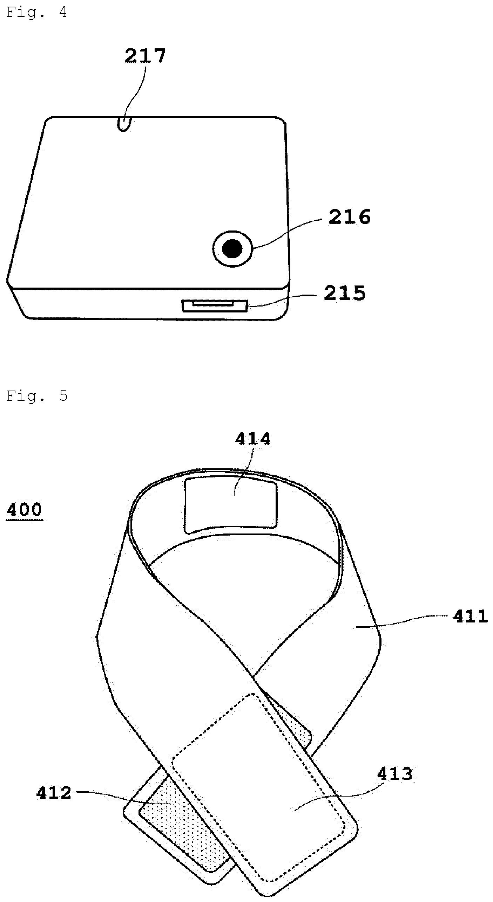

The auxiliary tool 400 may be any tool as long as it can assist the detection device 200 to be attached to the user. A band-shaped body having flexibility as shown in Fig. 5 is typically used, but a sticking plaster, a taping tape, a band, a bandage, a wound covering material, an adhesive tape, a supporter, or the like may be used. Furthermore, the auxiliary tool 400 is not necessarily configured as an individual body that is separable from the detection device 200, and a double-sided tape directly attached to the detection device 200, a wristwatch-like band, or the like can also be used as the auxiliary tool 400.

### 2. Configuration of Processing System 1

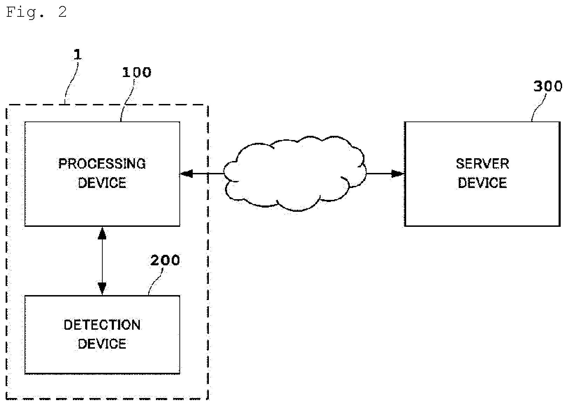

Fig. 2 is a schematic diagram of the processing system 1 according to an embodiment of the present disclosure. The processing system 1 includes the detection device 200 that is attached to the user and detects an output value during the user's exercise, and the processing device 100 that is communicably connected to the detection device 200 and processes the detected output value. Then, the processing system 1 is connected to a server device 300 via a network for wireless communication. The server device 300 includes a processor, a memory, a communication interface, and the like, and appropriately transmits and receives an instruction command, information, and the like necessary for processing in the processing device 100. Typically, in response to a request from the processing device 100, it transmits relevant information such as institution information and supplementary information stored in the server device 300, or receives update information therefor so as to update the information as needed and then store the information. Note that the details of the processing device 100 and the detection device 200 will be described later.

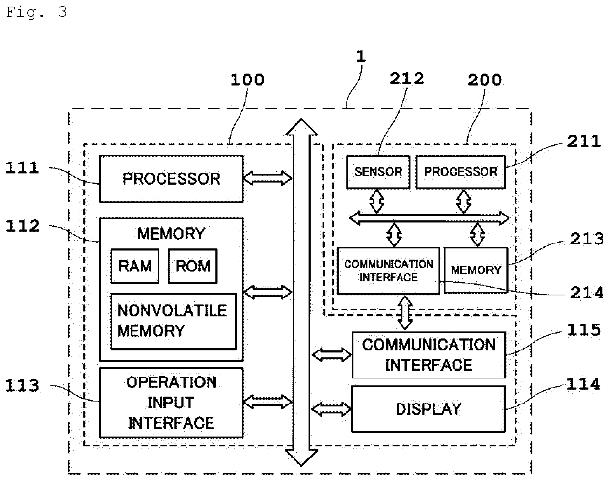

Fig. 3 is a block diagram illustrating a configuration of the processing system 1 according to the embodiment of the present disclosure. According to Fig. 3, the processing system 1 includes the processing device 100, and the detection device 200 communicably connected to the processing device 100 in a wireless or wired manner. The processing device 100 receives an operation input by the user and controls the detection of an output value during exercise by the detection device 200. In addition, the processing device 100 processes the output value detected by the detection device 200 to estimate the condition of the user during exercise or assist the estimation. Furthermore, the processing device 100 enables the user or the like to confirm an output value detected by the detection device 200, a value calculated on the basis of the output value, and information indicating a result of the estimation or the assistance therefor.

The processing system 1 includes the processing device 100 including a processor 111, a memory 112, an operation input interface 113, a display 114, and a communication interface 115, and the detection device 200 including a processor 211, a sensor 212, a memory 213, and a communication interface 214. These components are electrically connected to one another via a control line and a data line. Note that the processing system 1 does not necessarily include all of the components illustrated in Fig. 3, and can be configured by omitting a part of the components or by adding another component thereto. For example, the processing system 1 can include a battery for driving each component, a communication interface for transmitting a result of processing by the processing system 1 to the outside, or receiving an instruction command from the outside, and the like.

Note that the processing system 1 includes the processing device 100 and the detection device 200 as separable individual bodies. However, the present invention is not limited thereto, and the processing device 100 and the detection device 200 can be integrally configured as, for example, a wearable terminal device. Furthermore, the processing device 100 is not limited to a device configured as a single component, and in a case where at least a part of the processing is executed by another component (for example, a cloud server device or the like) connected thereto in a wired or wireless manner, a device including the other component may be referred to as a processing device 100.

First, the processing device 100 will be explained with reference to Fig. 3. The processor 111 functions as a control unit for controlling the other components in the processing system 1 on the basis of the program stored in the memory 112. The processor 111 controls driving of each component in the detection device 200 on the basis of the program stored in the memory 112, stores an output value received from the detection device 200 in the memory 112, and processes the stored output value. Specifically, the processor 111 executes, on the basis of a program stored in the memory 112, processing for receiving an instruction input to the operation input interface 113 by the user and turning on the detection device 200 to instruct the sensor 212 to perform detection, processing for receiving an output value transmitted from the detection device 200 via the communication interface 115 and storing the output value in the memory 112, processing for estimating the condition of the user's knee joint during exercise on the basis of the output value stored in the memory 112 or assisting the estimation, processing for outputting an output value after landing of the leg among the output values received from the detection device 200, processing for estimating a degree or a prognosis of knee osteoarthritis on the basis of the output value received from the detection device 200 or assisting the estimation, processing for outputting relevant information according to the output value received from the detection device 200, the condition of the knee joint, or the degree of knee osteoarthritis, processing for updating the relevant information at a predetermined timing, and the like. The processor 111 is mainly configured by one or a plurality of CPUs, but may be configured by combining a GPU or the like therewith appropriately.

The memory 112 includes a RAM, a ROM, a nonvolatile memory, a HDD, and the like, and functions as a storage unit. The memory 112 stores, as a program, instruction commands for various controls on the processing system 1 according to the present embodiment. Specifically, the memory 112 stores therein a program for causing the processor 111 to execute processing for receiving an instruction input to the operation input interface 113 by the user and turning on the detection device 200 to instruct the sensor 212 to perform detection, processing for receiving an output value transmitted from the detection device 200 via the communication interface 115 and storing the output value in the memory 112, processing for estimating the condition of the user's knee joint during exercise on the basis of the output value stored in the memory 112 or assisting the estimation, processing for outputting an output value after landing of the leg among the output values received from the detection device 200, processing for estimating a degree or a prognosis of knee osteoarthritis on the basis of the output value received from the detection device 200 or assisting the estimation, processing for outputting relevant information according to the output value received from the detection device 200, the condition of the knee joint, or the degree of knee osteoarthritis, processing for updating the relevant information at a predetermined timing, and the like. In addition to the program, the memory 112 stores therein an acceleration rate table, a user table, a KAM conversion table, an institution table, an auxiliary information table, and the like. Furthermore, in a case where machine learning is used for predicting a KAM value, estimating a condition of a knee joint, or the like, the memory 112 stores therein a trained KAM value estimation model. Note that, as the memory 112, it is possible to use a storage medium communicably connected to the outside or use such storage media in combination.

The operation input interface 113 functions as an operation input unit that receives the user's instruction input to the processing device 100 and the detection device 200. Examples of the operation input interface 113 include a "start button" for instructing the detection device 200 to start/end the detection, a "confirmation button" for performing various selections, a "back/cancel button" for returning to the previous screen or canceling an inputted confirmation operation, a cross key button for moving an icon or the like displayed on the display 114, an on/off key for turning on/off the power of the processing device 100, and the like. Note that, as the operation input interface 113, it is also possible to use a touch panel provided so to be superimposed on the display 114 and having an input coordinate system corresponding to a display coordinate system of the display 114. A method for detecting the user's instruction input through the touch panel may be any method such as a capacitance type or a resistive film type.

The display 114 functions as a display unit for displaying an output value detected by the detection device 200 or a value calculated on the basis of the output value, or for displaying a result of the estimation on the basis of the output value and the like. It is configured by a liquid crystal panel, but may be configured by an organic EL display, a plasma display, or the like instead of the liquid crystal panel.

The communication interface 115 functions as a communication unit for transmitting and receiving various commands related to the start of detection or the like, an output value detected by the detection device 200, and the like to and from the detection device 200 connected thereto in a wired or wireless manner, or for transmitting and receiving information to and from the server device 300. Examples of the communication interface 115 include various interfaces including a connector for wired communications such as USB or SCSI, a transmission/reception device for wireless communications such as LTE, Bluetooth (registered trademark), wifi, or infrared rays, various connection terminals for a printed mounting board or a flexible mounting board, and combinations thereof.

An example of the processing device 100 is a portable terminal device capable of wireless communication, typified by a smartphone. However, in addition to that, any device capable of executing processing according to the present disclosure, such as a tablet terminal, a laptop personal computer, a desktop personal computer, a feature phone, a personal digital assistant, and PDA can be suitably used.

Next, the detection device 200 will be explained. The processor 211 functions as a control unit for controlling the other components in the detection device 200 on the basis of the program stored in the memory 213. The processor 211 executes, specifically, processing for controlling the detection of an output value by the sensor 212, processing for storing the detected output value in the memory 213, processing for transmitting an output value stored in the memory 213 to the processing device 100 via the communication interface 214, and the like, on the basis of the program stored in the memory 213. The processor 211 is mainly configured by one or a plurality of CPUs, but may be configured by combining a GPU or the like therewith appropriately.

The memory 213 includes a R_AM, a ROM, a nonvolatile memory, a HDD, and the like, and functions as a storage unit. The memory 213 stores, as a program, instruction commands for various controls on the detection device 200 according to the present embodiment. Specifically, the memory 213 stores a program for causing the processor 211 to execute the processing for controlling the detection of an output value by the sensor 212, the processing for storing the detected output value in the memory 213, the processing for transmitting an output value stored in the memory 213 to the processing device 100 via the communication interface 214, and the like. In addition to the program, the memory 213 stores therein an output value detected by the sensor 212. Note that, as the memory 112, it is possible to use a storage medium communicably connected to the outside or use such storage media in combination.

The sensor 212 is driven according to an instruction from the processor 211, and functions as a detection unit for detecting an output value during the user's exercise. As one example, an acceleration sensor is used as the sensor 212. The acceleration sensor detects a change ratio of a movement amount (velocity) per unit time. The types thereof include a capacitance type, a piezo type, and a heat detection type, and any of these can be suitably used. It is preferable that the acceleration sensor can detect the acceleration rate at least in a horizontal direction, and can further detect the acceleration rate in a vertical direction and/or the acceleration rate in a depth direction. In addition, as the sensor 212, it is possible to use a gyro sensor in combination with an acceleration sensor. In this case, it is possible to obtain three output values, that is, the angular velocity with respect to an axis in the horizontal direction, the angular velocity with respect to an axis in the vertical direction, and the angular velocity with respect to an axis in the depth direction, by the gyro sensor. That is, in addition to a total of three acceleration rates in the horizontal direction, the vertical direction, and the depth direction, the three angular velocities (that is, output values of six axes in total) are available. Note that, in addition to this example, sensors capable of detecting the exercise of the user 10, in particular, movements including bending and stretching or shaking of the knee, such as a geomagnetic sensor and an expansion/contraction sensor, can be appropriately used in combination.

The communication interface 214 functions as a communication unit for transmitting and receiving various commands related to the start of detection or the like, an output value detected by the detection device 200, and the like to and from the processing device 100 connected thereto in a wired or wireless manner. Examples of the communication interface 214 include various interfaces including a connector for wired communications such as USB or SCSI, a transmission/reception device for wireless communications such as LTE, Bluetooth (registered trademark), wifi, or infrared rays, various connection terminals for a printed mounting board or a flexible mounting board, and combinations thereof.

Fig. 4 illustrates an external appearance of the detection device 200 according to the embodiment of the present disclosure. Specifically, it illustrates an example of an external appearance in a case where an acceleration sensor is used as the sensor 212 in the detection device 200. According to Fig. 4, the detection device 200 has, on an upper surface thereof, a power switch 216 for switching on/off a power source for the detection device 200. In addition, the detection device 200 includes a USB terminal 215 as an example of the communication interface 214. Furthermore, the detection device 200 includes an indicator 217 in order to give notice of a driving state such as abnormality.

As one example, the detection device 200 is attached to or around the user's knee (typically, below the knee on a front face) using the auxiliary tool 400. Fig. 5 illustrates an external appearance of the auxiliary tool 400 according to the embodiment of the present disclosure. The auxiliary tool 400 has such a length in a short direction that corresponds to a length of the detection device 200 in the short direction, and such a length in a longitudinal direction that is sufficient to cover the periphery of the user's leg. The auxiliary tool 400 is typically formed by a sheet-like material having flexibility. The auxiliary tool 400 has a pair of fixing members 412 and 413 at both ends thereof. Examples of such fixing members 412 and 413 include hook-and-loop fasteners, but it is possible to use any other fixing members capable of joining end portions together, such as buttons and adhesive tapes.

The auxiliary tool 400 includes a bag member 414 for accommodating the detection device 200 therein at substantially the center thereof in the longitudinal direction. The bag member 414 has a size corresponding to the size of the detection device 200. Therefore, by inserting the detection device 200 into the bag member 414 of the auxiliary tool 400 and attaching the auxiliary tool 400 having the detection device 200 inserted therein to the leg, it is possible to prevent the detection device 200 from being displaced inside the auxiliary tool 400 due to the exercise, and detect only the vibration generated from the exercise appropriately. That is, the bag member 414 is used to position the detection device 200 in a more reliable manner.

Note that such an auxiliary tool 400 is merely an example. As mentioned above, a sticking plaster, a taping tape, a band, a bandage, a wound covering material, an adhesive tape, a supporter, or the like may be used. Furthermore, the auxiliary tool 400 is not necessarily configured as an individual body that is separable from the detection device 200, and a double-sided tape directly attached to the detection device 200, a wristwatch-like band, or the like can also be used as the auxiliary tool 400.

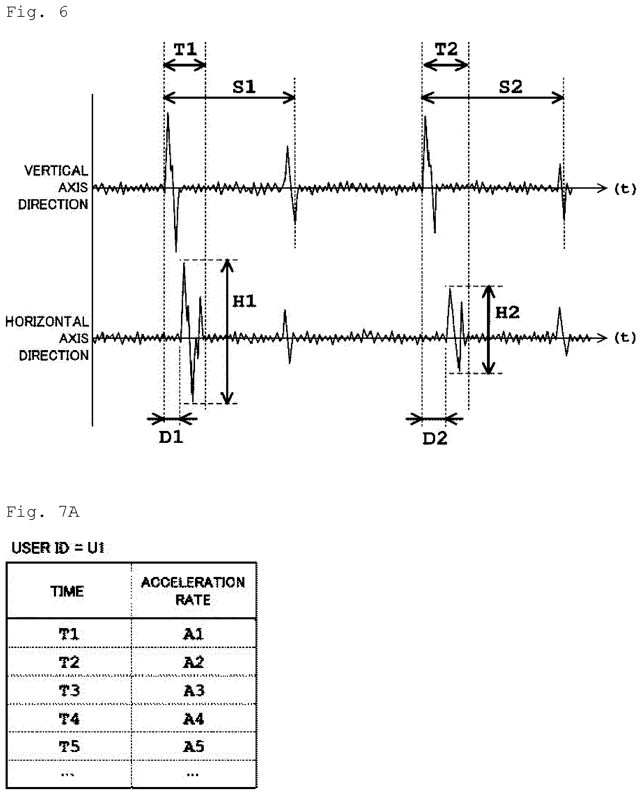

Fig. 6 illustrates one example of an output value detected by the detection device 200 according to the embodiment of the present disclosure. Specifically, it illustrates an example in which an acceleration sensor is used as the sensor 212 in the detection device 200 and is attached below the user's knee on a front face (for example, only the left leg) so as to detect both the acceleration rate in the vertical direction (vertical axis direction in Fig. 6) and the acceleration rate in the horizontal direction (horizontal axis direction in Fig. 6) during walking exercise. Here, generally, during walking exercise, once a heel portion of one leg (for example, the left leg) of the user reaches the ground, the knee joint in an extended condition starts to bend, and the user's body including the knee starts to move in a traveling direction. Thereafter, the leg that has reached the ground at the heel portion, achieves a condition in which substantially the entire sole reaches the ground. Next, when the user takes further steps, the leg starts to gradually separate from the ground from the heel portion, and finally, when the user moves so as to kick the ground with the fingertip, the fingertip completely separates from the ground. On the other hand, as for the leg on the opposite side (for example, the right leg), when the heel portion of the left leg is about to reach the ground, the fingertip starts to separate from the ground, and when the fingertip of the left leg starts to separate from the ground, the heel portion reaches the ground. As discussed above, the walking exercise is performed by periodically repeating a stance phase from landing of the heel portion to the separation of the fingertip and a separation period from the separation of the fingertip to the landing of the heel portion.

In Fig. 6, by focusing on the acceleration rate in the vertical direction (that is, the vertical axis direction), a first peak of the acceleration rate is detected when the heel of one leg reaches the ground, and then a next peak of the acceleration rate is detected when the heel of the leg on the opposite side reaches the ground. Therefore, a period between the rising of the first peak and the falling of the next peak can be set as a stance phase S1. Then, these two peaks are periodically detected each time the stance phase such as a stance phase S2 is coming.

At this time, by focusing on the acceleration rate in the horizontal direction (that is, the horizontal axis), a first peak is detected within a predetermined period after the start of the stance phase. For example, in the case of a disease such as knee osteoarthritis, a thrust occurs in the horizontal direction once the stance phase starts and a load is applied to the knee joint. Therefore, it is possible to effectively estimate the condition of the knee joint and the condition of knee osteoarthritis by detecting the movement in the horizontal direction after the start of the stance phase.

Here, there is a KAM (external knee adduction moment) value as an index for evaluating a degree or a prognosis of knee osteoarthritis. It is considered that, when the KAM value reaches or exceeds a certain value, the risk of progress of knee osteoarthritis becomes high after a lapse of a predetermined period, and when the KAM value becomes equal to or less than another certain value, the risk of progress is low (document 1: T Miyazakira, Dynamic load at baseline can predict radiographic disease progression in medial compartment knee osteoarthritis, Ann Rheum Dis., 2002, No. 61, pp. 617-622, document 2: Kim L Bennell et all, Higher dynamic medial knee load predicts greater cartilage loss over 12 months in medial knee osteoarthritis, Ann Rheum Dis., 2011, No. 70, pp. 1770-1774, document 3: Nicholas M. Brisson, Baseline Knee Adduction Moment Interacts With Body Mass Index to Predict Loss of Medial Tibial Cartilage Volume Over 2.5 Years in Knee Osteoarthritis, JOURNAL OF ORTHOPAEDIC RESEARCH, 2017, NOVEMBER, pp. 2476-2483). Here, there is a certain correlation between the KAM value and the acceleration rate in the horizontal direction detected by the acceleration sensor attached to the knee or around the knee. Therefore, by calculating the KAM value from the detected acceleration rate in the horizontal direction, it is possible to estimate a degree or a prognosis of knee osteoarthritis or assist the estimation. Specifically, values of peak widths H1 and H2 of the acceleration rate in the horizontal direction (that is, the horizontal axis) detected within predetermined periods T1 and T2 after the start of the stance phases S1 and S2 specified by the acceleration rate in the vertical direction (that is, the vertical axis) are calculated, and the KAM value is estimated on the basis of these values.

In addition, it has been found that, in a case where the user suffers from a symptom of knee osteoarthritis, time taken until a peak of the acceleration rate in the horizontal direction is detected is shorter and the number of peaks detected within a predetermined period after the start of the stance phase is larger as compared with users who present no symptom thereof. Therefore, it is possible to use times D1 and D2 taken until a peak of the acceleration rate in the horizontal direction is detected after the start of the stance phases S1 and S2, and the number of peaks detected within the predetermined periods T1 and T2, in place of or in combination with the peak widths H1 and H2, as an index for evaluating a degree or a prognosis of knee osteoarthritis.

As discussed above, by using output values (acceleration rate in the vertical direction and acceleration rate in the horizontal direction) detected by the detection device 200 illustrated in Fig. 6, it is possible to estimate a KAM value and evaluate a condition of a human's knee joint during exercise, specifically, a degree or a prognosis of knee osteoarthritis. Note that the acceleration rate in the vertical direction, among output values detected by the detection device 200, is used to specify the exercise cycle, that is, the stance phase for the human. Therefore, it is also possible to use another numerical value as long as it is synchronized with a value of the acceleration rate in the horizontal direction and the stance phase can be specified, and a value of the acceleration rate in the vertical direction is not necessarily required.

Note that, in the present disclosure, any of the following two values can be used as the "KAM value". There is a two-dimensional curve (KAM value curve) in which time is plotted on the horizontal axis and KAM values calculated at each time are plotted on the vertical axis. At this time, the highest peak value (KAM peak value) detected during the stance phase can be used as the first KAM value. It is possible to reflect a value at a moment when the largest force is applied to the knee joint during the stance phase, on this KAM peak value. As the second KAM value, an area value (KAM area value) between a KAM value curve and the horizontal axis (straight line) during the stance phase can be used. It is possible to reflect a value of a whole load applied to the knee joint during the stance phase, on this KAM area value. In fact, the KAM peak value is used for evaluating a degree or a prognosis of knee osteoarthritis in document 1 listed above, and the KAM area value is used for evaluating a degree or a prognosis of knee osteoarthritis in documents 2 and 3 listed above.

### 3. Information Stored in Processing Device 100

Fig. 7A illustrates one example of an acceleration rate table stored in the processing device 100 according to the embodiment of the present disclosure. The acceleration rate table is prepared for each user, and is stored in association with user ID information for specifying the user. Fig. 7A illustrates, as an example, an acceleration rate table for a user having user ID information of "U1". According to Fig. 7A, the acceleration rate table stores therein acceleration rate information in association with time information. The "time information" is information for specifying a time at which each acceleration rate is measured in the detection device 200. This information may be information about specific date and time with a timer included in the detection device 200, or may be time elapsed since the start of measurement, or the like. The "acceleration rate information" is information indicating a specific value of the acceleration rate detected in the corresponding time information. Once the "time information" and the "acceleration rate information" are both detected in the detection device 200, the information is transmitted from the detection device 200 and is stored in the memory 112 of the processing device 100 which has received the information. Note that, in Fig. 7A, the acceleration rate in the horizontal direction is typically stored, as the acceleration rate information, in association with each piece of the time information. However, the present invention is not limited thereto, and in addition to the acceleration rate in the horizontal direction, the acceleration rate in the vertical direction may also be stored in association with each piece of the time information.

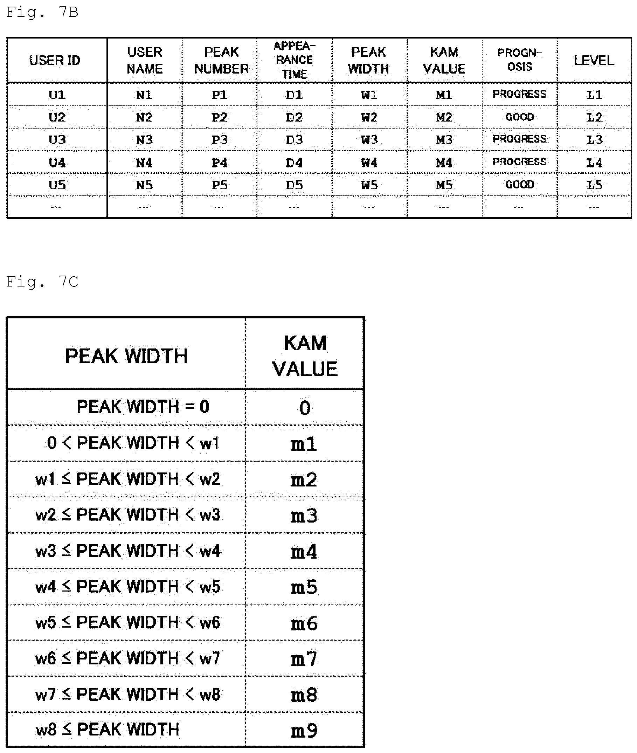

Fig. 7B illustrates one example of a user table stored in the processing device 100 according to the embodiment of the present disclosure. According to Fig. 7B, the user table stores user name information, peak number information, appearance time information, peak width information, KAM value information, prognosis information, and level information in association with user ID information. The "user ID information" is information generated each time the detection device 200 is attached to the user to be measured for the acceleration rate and the user is newly registered, and is information unique to each user and used to specify each user. The "user name information" is information indicating the name of the user to be displayed on, for example, the display 114 in the processing device. The "peak number information" is information specified on the basis of the acceleration rate information stored in the acceleration rate table stored for each piece of the user ID information, and is information indicating the number of peaks of the acceleration rate in the horizontal direction detected within a predetermined period (for example, T1 and T2) in Fig. 6. The "appearance time information" is information specified on the basis of the acceleration rate information stored in the acceleration rate table stored for each piece of the user ID information, and is information indicating time taken from the start during the stance phase (for example, S1 and S2) until a peak of an acceleration rate in the horizontal direction is detected (for example, D1 and D2) in Fig. 6. The "peak width information" is information specified on the basis of the acceleration rate information stored in the acceleration rate table stored for each piece of the user ID information, and is information indicating a peak width of an acceleration rate in the horizontal direction detected after the start during the stance phase (for example, S1 and S2) and within a predetermined period (for example, D1 and D2) in Fig. 6. The "KAM value information" is information estimated on the basis of the peak width information, and is information used to evaluate the prognosis of knee osteoarthritis. The "prognosis information" is information specified on the basis of the KAM value information, and is information indicating a result of estimation of the prognosis of knee osteoarthritis. The "level information" is information indicating the current condition of the knee estimated on the basis of at least one of the peak number information, the appearance time information, the peak width information, and a combination thereof, specifically, a result of estimation of the degree of knee osteoarthritis.

Fig. 7C illustrates one example of a KAM conversion table stored in the processing device 100 according to the embodiment of the present disclosure. According to Fig. 7C, the KAM conversion table stores therein the KAM value information in association with the peak width information. The "peak width information" is information indicating a numerical range of each peak width. The "KAM value information" is information indicating an estimated value of a KAM value corresponding to the numerical range of each peak width. That is, an appropriate numerical range is specified in the KAM conversion table in Fig. 7C on the basis of the peak width information stored in Fig. 7B, the KAM value information corresponding to the numerical range is calculated as an estimated value of the KAM value. Then, the estimated KAM value is stored as the KAM value information in Fig. 7B.

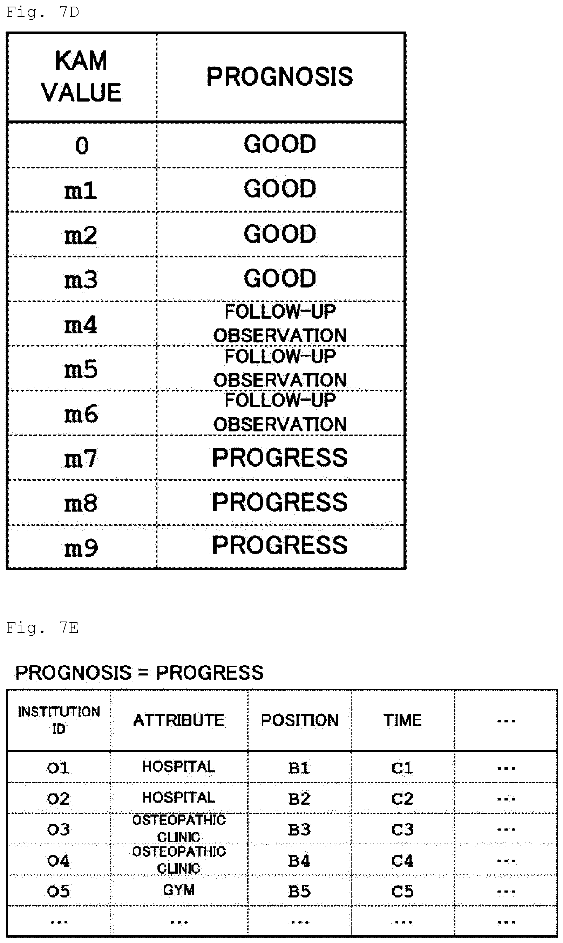

Fig. 7D illustrates one example of a prognosis conversion table stored in the processing device 100 according to the embodiment of the present disclosure. According to Fig. 7D, the prognosis information is stored in the prognosis conversion table in association with the KAM value information estimated by using the KAM conversion table in Fig. 7C. As described above, the "KAM value information" is information indicating each KAM value estimated by using the KAM conversion table in Fig. 7C. The "prognosis information" is information indicating a result of the evaluation of the prognosis corresponding to each KAM value. That is, an evaluation result of the prognosis is calculated from the KAM value information stored in Fig. 7B, with reference to a prognosis conversion table in Fig. 7D. In the prognosis information, "good" means that the prognosis is good, "follow-up observation" means that a follow-up observation for periodically confirming a degree of progress of knee osteoarthritis is required for the prognosis, and "progress" means that the progress of knee osteoarthritis is predicted. Note that, in the present embodiment, the prognosis is evaluated in three stages, but it is naturally possible to perform the evaluation in further divided stages such as "fast progress" and "slow progress". The prognosis information evaluated by using the prognosis conversion table is stored as the prognosis information in Fig. 7B.

Fig. 7E illustrates one example of an institution table stored in the processing device 100 according to the embodiment of the present disclosure. The institution table is prepared in association with each piece of the prognosis information stored in Fig. 7D. That is, different institution tables are stored according to the prognosis information stored in Fig. 7D, and various pieces of information are stored in each institution table. The example illustrated in Fig. 7E shows one example of an institution table to be referred to when "progress" is selected as the prognosis information in Fig. 7B. The auxiliary information table stores therein information related to a medical institution or a doctor capable of executing at least either one of medical examination and medical treatment, or information related to an institution that supports the prognosis. Specifically, according to Fig. 7E, attribute information, position information, time information, and the like are stored in association with institution ID information. The "institution ID information" is information generated each time a new institution serving as a source of information is registered, and is information unique to each institution and information for specifying each institution. The "attribute information" is information indicating the type of an institution specified by each piece of the institution ID information. For example, "hospital" means that the institution is a hospital, "osteopathic clinic" means that the institution is an osteopathic clinic, and "gym" means that the institution is an athletic gym. The "position information" is information indicating the position of an institution specified by each piece of the institution ID information. Examples of the position information include coordinate information such as latitude/longitude with a predetermined position as an origin, address information, and the like. The time information is information indicating an opening (hospital opening) time, a regular holiday (non-consultation day), and the like of an institution specified by each piece of the institution ID information. These pieces of information are updated as needed at a timing when information is regularly or newly inputted. Note that, although the attribute information and the like are mentioned as examples of information stored in the institution table, it is obviously possible to store other kinds of information as well. For example, it is possible to store various items such as information about a name of a doctor in charge or a main trainer, information about subjects of medical treatment, information about medical treatment achievement, evaluation information, and information about photographs of an external appearance or an internal appearance of an institution. In addition, although not particularly illustrated, similarly to the prognosis information, it is also possible to further store the institution table in association with each piece of the level information. That is, it is also possible to store different institution tables according to the level information stored in Fig. 7D, and store various pieces of information in each institution table.

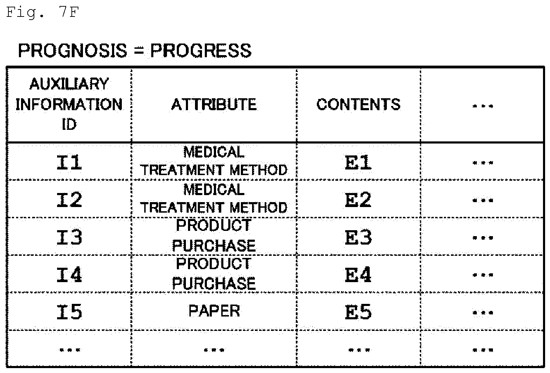

Fig. 7F illustrates one example of an auxiliary information table stored in the processing device 100 according to the embodiment of the present disclosure. The auxiliary information table is prepared in association with each piece of the prognosis information stored in Fig. 7D. That is, different auxiliary information tables are stored according to the level information stored in Fig. 7D, and various pieces of information are stored in each auxiliary information table. The example illustrated in Fig. 7F shows one example of an auxiliary information table to be referred to when "progress" is selected as the prognosis information in Fig. 7B. The auxiliary information table stores therein information for assisting doctors executing at least either one of medical examination and medical treatment, or information for assisting users (patients) to purchase products. Specifically, according to Fig. 7F, attribute information, content information, and the like are stored in association with auxiliary information ID information. The "auxiliary information ID information" is information generated each time information related to the prognosis of progressive knee osteoarthritis is registered, and is information unique to each piece of information and information for specifying each piece of information. The "attribute information" is information indicating the type of information specified by each piece of the auxiliary information ID information. For example, "medical treatment method" means that the information is information related to a medical treatment method, "product purchase" means that the information is used to purchase products such as a beneficial instrument for the purpose of suppressing the degree of progress of knee osteoarthritis, and "paper" means a paper related to the prognosis of knee osteoarthritis. The "content information" is information indicating specific contents of information specified by each piece of the auxiliary information ID information. For example, it stores: a link to a web page introducing a medical treatment method, a specific medical treatment method therefor, and the like in a case of a medical treatment method; detailed information about a product, a supplier's address therefor, and the like in a case of a product purchase; and information about specific contents, an author's name, an affiliated institution, and the like in a case of a paper. These pieces of information are updated as needed at a timing when information is regularly or newly inputted. Note that, although the attribute information and the like are mentioned as examples of information stored in the auxiliary information table, it is obviously possible to store other kinds of information as well. For example, it is possible to store various contents such as other users' experiences and citation information/quotation information. In addition, although not particularly illustrated, similarly to the prognosis information, it is also possible to further store the auxiliary information table in association with each piece of the level information. That is, it is also possible to store different auxiliary information tables according to the level information stored in Fig. 7D, and store various pieces of information in each auxiliary information table.

### 4. Processing Flow Executed in Processing Device 100

### [Processing related to mode selection]

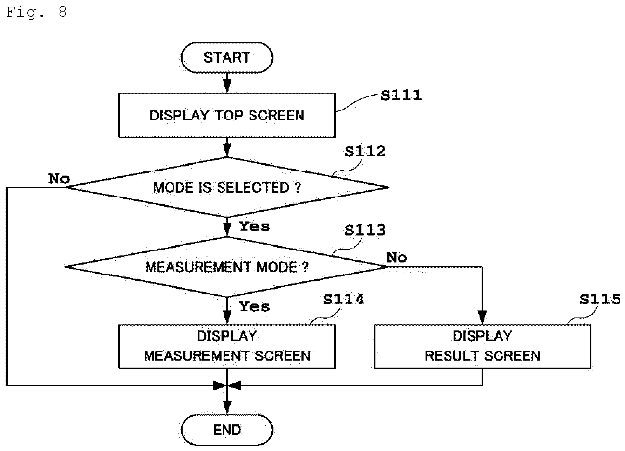

Fig. 8 illustrates a processing flow executed in the processing device 100 according to the embodiment of the present disclosure. Specifically, Fig. 8 illustrates a processing flow to be executed by the processor 111 at a predetermined cycle after a program according to the embodiment of the present disclosure is activated in the processing device 100.

First, when receiving an interrupt signal indicating that an instruction input for activating the program has been received by the operation input interface 113, the processor 111 displays a top screen on the display 114 (5111). Although not particularly illustrated, the top screen includes an icon for a measurement mode for measuring an output value from the user in the detection device 200 and an icon for transitioning to a result display mode for displaying the measurement result. Thereafter, the processor 111 determines whether or not a mode has been selected on the basis of the interrupt signal indicating that the operation input with respect to an icon by an operator is received through the operation input interface 113 (S112). In a case where it is determined that no mode has been selected, a state where the top screen is displayed is maintained as it is, and the processing flow ends.

On the other hand, when it is determined that a mode has been selected, the processor 111 determines whether the measurement mode has been selected on the basis of coordinates at which the operation input by the operator has been made (S113). Then, when it is determined that the selected mode is the measurement mode, the processor 111 controls the display 114 to display a measurement screen, and ends the processing flow. On the other hand, when it is determined that the selected mode is not the measurement mode, the processor 111 controls the display 114 to display a result screen, and ends the processing flow.

Note that, although the measurement screen is not particularly illustrated, the measurement screen displays a region in which user name information and user ID information of the user are inputted or selected, a start button icon for starting the measurement, and the like (not particularly illustrated).

### [Processing related to measurement start]

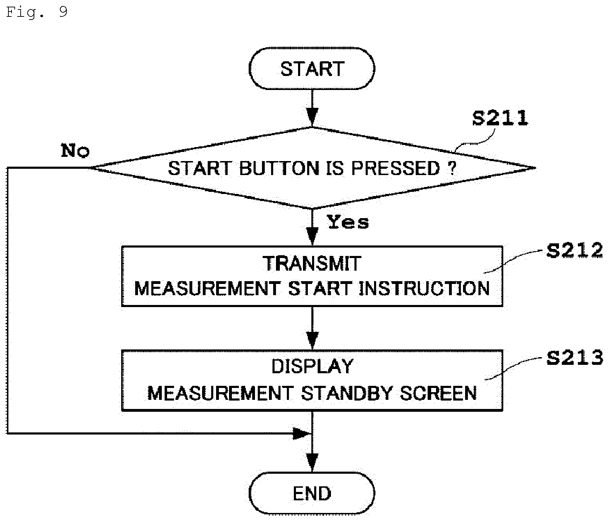

Fig. 9 illustrates a processing flow executed in the processing device 100 according to the embodiment of the present disclosure. Specifically, Fig. 9 illustrates a processing flow to be executed by the processor 111 at a predetermined cycle after the measurement mode is selected and the measurement screen is displayed in Fig. 8. Note that, although not particularly illustrated in Fig. 9, before the processing illustrated in Fig. 9, a measurer or a user inputs or selects the user name information and the user ID information on the measurement screen, and the processor 111 stores the inputted or selected information in the memory 112. In addition, the detection device 200 is mounted in advance to a lower portion of the knee of one leg of the user by the auxiliary tool 400, and all preparations for starting the user's exercise (for example, walking) are completed. In the description of the processing flow from Fig. 9 onward, for convenience of explanation, a case where the detection device 200 is mounted to the knee of only one of the legs will be described.

According to Fig. 9, the processor 111 determines whether the operation input with respect to the measurement button icon by the operator has been received through the operation input interface 113 (S211). Then, when it is determined that the operation input with respect to the start button icon has been received, the processor 111 performs control to transmit a measurement start instruction signal for instructing the detection device 200 to start measurement via the communication interface 115 (S212). Thereafter, the processor 111 performs control to display a measurement standby screen on the display 114 (S213). Note that, although not particularly illustrated, an end button icon for ending the measurement or the like is displayed on the measurement standby screen.

Here, the processing on the detection device 200 side will be explained. When receiving a measurement start signal via the communication interface 214 after being mounted to the user, the processor 211 of the detection device 200 drives the sensor 212 to start the detection of the acceleration rate at a predetermined cycle (Fig. 7A). Then, the processor 211 stores, as needed in the memory 213, the detected acceleration rate as an output value in association with the detection time. Then, the processor 211 executes this processing until a measurement end instruction signal is received from the processing device 100.

Note that a case where the measurement start instruction signal is transmitted upon the detection of the pressing of the start button displayed on the display 114 has been explained. However, the present invention is not limited thereto, and the processor 111 may perform the transmission upon the detection of the pressing of a start button provided as a physical key in the operation input interface 113. Control may be also performed such that, for example, when the operation of pressing the power switch 216 of the detection device 200 is received, the detection device 200 transmits the measurement start signal to the processing device 100, and then the processor 111 displays the measurement standby screen.

The processing flow related to the measurement start is ended as described above.

### [Processing performed during measurement standby]

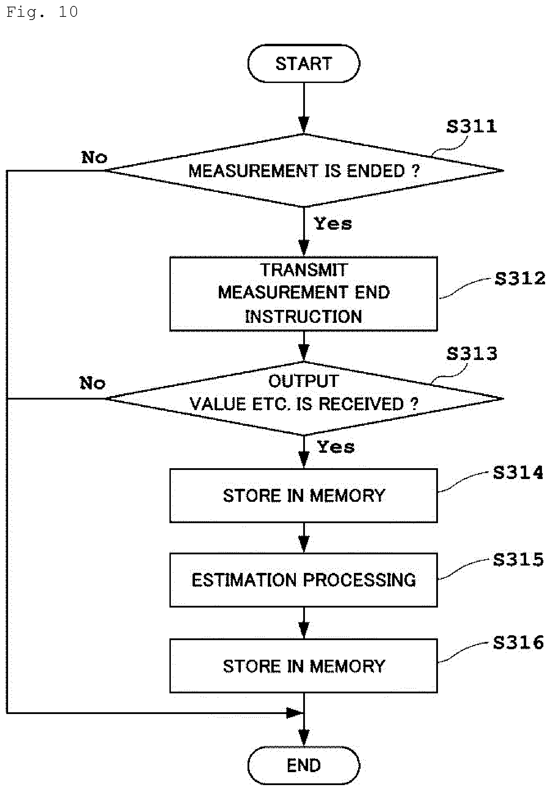

Fig. 10 illustrates a processing flow executed in the processing device 100 according to the embodiment of the present disclosure. Specifically, Fig. 10 illustrates a processing flow to be executed by the processor 111 at a predetermined cycle after the measurement standby screen is displayed in Fig. 9.

According to Fig. 10, the processor 111 determines whether the operation input with respect to the end button icon by the operator has been received through the operation input interface 113 and the measurement has ended (S311). Then, when it is determined that the measurement has ended, the processor 111 performs control to transmit the measurement end instruction signal for instructing the detection device 200 to end measurement via the communication interface 115 (S312).

Here, in the detection device 200 that has received the measurement end instruction signal from the processing device 100 via the communication interface 214, the processor 211 controls the sensor 212 to end the detection of the acceleration rate. Then, the processor 211 performs control to transmit the output value and the time information stored in the memory 213 to the processing device 100 via the communication interface 214 until the end.

In the processing device 100, it is determined whether the processor 111 has received the output value and the time information from the detection device 200 via the communication interface 115 (S313). When it is determined that the reception has been performed, the processor 111 stores the output value (acceleration rate information) received in association with the user ID information which has been inputted or selected on the measurement screen, in the acceleration rate table of the memory 112 in association with the time information (S314). Next, the processor 111 performs various kinds of estimation processing on the basis of the output value stored in the memory 112 (S315). The details of this processing will be described later. Then, the processor 111 stores various types of information obtained in the course of the estimation processing, in the user table of the memory 112 in association with the user ID information (S316).

Note that a case where the measurement end instruction signal is transmitted upon the detection of the pressing of the end button displayed on the display 114 has been explained. However, the present invention is not limited thereto, and the processor 111 may perform the transmission upon the detection of the pressing of an end button provided as a physical key in the operation input interface 113. It may be also configured such that, for example, when the operation of pressing the power switch 216 of the detection device 200 is received, the detection device 200 ends the measurement and transmits the output values and the like to the processing device 100.

The processing flow performed during the measurement standby is ended as described above.

### [Estimation process]

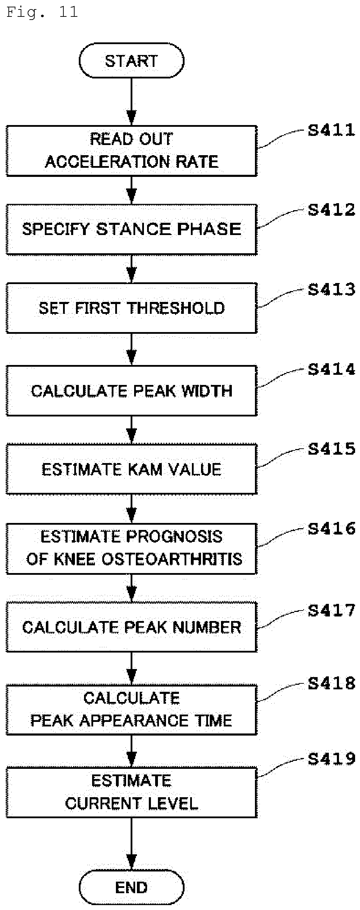

Fig. 11 illustrates a processing flow executed in the processing device 100 according to the embodiment of the present disclosure. Specifically, Fig. 11 illustrates a specific processing flow of the estimation processing performed in S315 in Fig. 10.

According to Fig. 11, the processor 111 first reads out acceleration rate information associated with the user ID information which has been inputted or selected on the measurement screen from the acceleration rate table stored in the memory 112 (S411). Fig. 6 is an example of a curve generated on the basis of the acceleration rate information which has been read out. Therefore, in the following description, an explanation will be made with reference to Fig. 6.

Next, the processor 111 specifies each stance phase on the basis of the acceleration rate information which has been read out (S412). Specifically, a first peak of the acceleration rate is detected from the acceleration rate in the vertical direction (that is, the vertical axis direction in Fig. 6) among the acceleration rates which have been read out. This first peak is a peak detected when the leg to which the detection device 200 is mounted reaches the ground. Note that this peak may be determined as the first peak by the processor 111 when the acceleration rate exceeding a predetermined threshold is detected, or may be specified by displaying an acceleration rate curve illustrated in Fig. 6 on the display 114 and receiving an operation input from an operator. Hereinafter, processing related to the detection of a peak is performed in a similar manner.

Next, after the first peak is detected, a time during which only noise components are detected passes, and then the second peak is detected. This second peak is a peak detected when the leg on a side opposite to the leg to which the detection device 200 is mounted reaches the ground. Therefore, the processor 111 determines that a period from the rising of the first peak until the falling of the second peak is detected, is determined as the stance phase S1. Note that, hereinafter, although a period between the rising of a third peak and the falling of a fourth peak is specified as the stance phase S2, fifth and subsequent peaks may be further detected to further specify stance phases.

Next, the processor 111 sets a first threshold (time) for each of the stance phases S1 and S2 which have been specified (S413). Specifically, with respect to the period specified as the stance phase S1, a time from the start thereof until a predetermined period T1 has elapsed, is set as the first threshold. A period corresponding to 40%, more preferably a period corresponding to 25%, of a period of the stance phase S1 is set as the predetermined period T1. Similarly, the first threshold is also set in the period of the stance phase S2. Note that, in the setting of the first threshold, the ratio thereof with respect to the stance phases S1 and S2 is used, but the present invention is not limited thereto. A predetermined fixed value (for example, 50 ms after the start of the stance phase) may be used as the first threshold.

Next, the processor 111 detects a peak of the acceleration rate in the horizontal direction (that is, the horizontal axis direction in Fig. 6) in the period T1 until the set first threshold (time), calculates a peak width thereof, and stores the calculated peak width in association with the user ID information in the peak width information of the user table (S414). Specifically, a peak of the acceleration rate in the horizontal direction is detected during a period from the start of the stance phase S1 until the first threshold is exceeded. Then, a difference between the maximum value and the minimum value of the detected peak is calculated as the peak width H1. Similarly, the peak width H2 of the acceleration rate in the horizontal direction in the stance phase S2 is also calculated. Then, an average value of the calculated peak widths H1 and H2 is used as peak value information in S414. Note that, although the average value is used in the present embodiment, either one of a larger value or a smaller value may be used, or both values may be used.

Next, the processor 111 refers to the KAM conversion table (Fig. 7C) stored in the memory 112, estimates a KAM value from the value of the peak width calculated in S414, and stores the estimated KAM value in association with the user ID information as the KAM value information of the user table (S415). For example, when the peak width calculated in S414 is w6 or more and less than w7, the estimated KAM value is m7.

Then, the processor 111 refers to the prognosis conversion table (Fig. 7D) stored in the memory 112, estimates the prognosis of knee osteoarthritis from the KAM value stored in S415, and stores the estimated prognosis in association with the user ID information in the prognosis information of the user table (S416). For example, when the KAM value stored in S415 is m7, the estimated prognosis is "progress".

Next, the processor 111 calculates the number of peaks detected in the acceleration rate in the horizontal direction during a period from the start of the stance phases S1 and S2 until the first threshold, and stores the calculated number of peaks in association with the user ID information in the peak number information of the user table (S417). Specifically, in the example in Fig. 6, since there are two peaks exceeding the predetermined threshold (output value) during the period T1 until the first threshold is exceeded in the stance phase S1, the number of peaks is calculated as "2". Similarly, since there are two peaks exceeding the predetermined threshold (output value) during the period T2 until the first threshold is exceeded in the stance phase S2, the number of peaks is calculated as "2". Then, an average value of the calculated number of peaks is used as peak number information in S417. Note that, although the average value is used in the present embodiment, either one of a larger number or a smaller number may be used, or both numbers may be used.

Next, in the stance phases S1 and S2, the processor 111 calculates the appearance time of the peak that is firstly detected in the acceleration rate in the horizontal direction, and stores the calculated appearance time in association with the user ID information in the appearance time information of the user table (S418). Specifically, in the example in Fig. 6, the period D1 and the period D2 are calculated as appearance times, respectively, by subtracting the start time of the stance phase S1 from a time when the first peak is detected in the stance phase S1, and by subtracting the start time of the stance phase S2 from the time when the first peak is detected in the stance phase S2.

Here, the peak width calculated in S414 is a numerical value related to the magnitude of a horizontal deviation of the knee joint. In addition, as knee osteoarthritis progresses, a horizontal wobble of the knee joint increases, and thus, the number of peaks calculated in S417 is a numerical value related to the degree of horizontal wobble. Furthermore, in a symptom of the knee such as knee osteoarthritis, the thinner the cartilages of the knee joint become, the more the symptom progresses, and the thinner the cartilages become, the faster the vibration is transmitted to the knee joint, and thus, the peak appearance time calculated in S418 is information related to the thickness of the cartilages. Therefore, each piece of information about the peak width calculated in S414, the number of peaks calculated in S417, and the peak appearance time calculated in S418 for the acceleration rate in the horizontal direction is useful information for estimating the current condition of the knee, for example, the level of knee osteoarthritis. Therefore, the processor 111 estimates the level of the current condition of the knee using these pieces of information stored in the memory, and stores the estimated value in association with the user ID information in the user table (S419). As an example of the estimation, each piece of the obtained information is scored on the basis of a classification table set in advance. Thereafter, each piece of the obtained information is multiplied by a predetermined weighting coefficient, and a sum of the obtained weighted numerical values is calculated. Then, the level of the condition of the knee is estimated on the basis of the sum. Note that this example is one example as described above, and the other methods can be used. For example, a predetermined threshold is set in advance for each of the peak width, the number of peaks, and the peak appearance time, and when any one of the numerical values exceeds the threshold, or when two of the numerical values exceed the threshold, or when all of the three numerical values exceed the threshold, the current condition of the knee may be estimated as knee osteoarthritis.

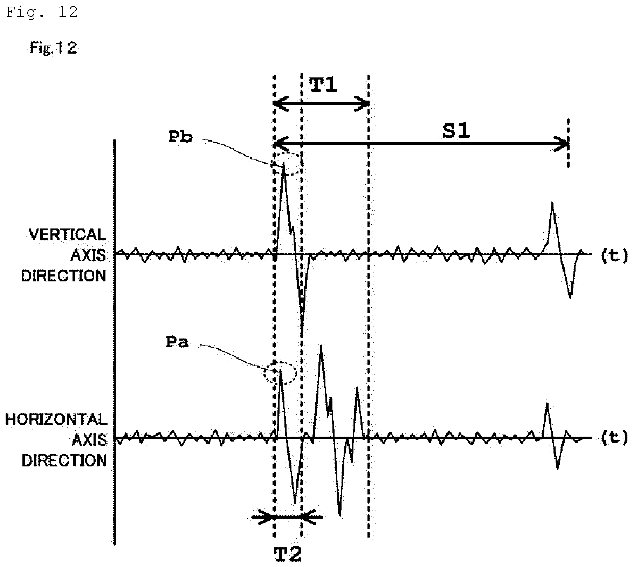

Here, Fig. 12 illustrates one example of an acceleration rate detected by the detection device 200 according to the embodiment of the present disclosure. According to Fig. 12, a plurality of peaks of the acceleration rate in the horizontal axis direction, that is, in the horizontal direction are detected during the period T1 in the stance phase S1. One of them is a peak Pa detected immediately after the start of the stance phase. However, in the present embodiment, the peak Pa is determined as noise, and will be "ignored" in each processing in S414, S417, and S418. When the leg to which the detection device 200 is mounted reaches the ground at the beginning of the stance phase, the vibration of the entire leg in the horizontal direction due to the landing may be detected. This vibration does not result from the horizontal deviation of the knee joint itself caused by knee osteoarthritis, and needs to be specified as noise.

Specifically, when the stance phase S1 is specified in S412, the processor 111 determines whether respective peaks detected in S414, S417, and S418 are peaks detected during the period T2 from the start of the stance phase S1 until the second threshold (time) is exceeded. Then, if the peak has been detected during that period, the processor 111 determines that the peak is noise and does not set the peak as a detection target in each processing. Note that, in processing related to the noise specification, for example, a period corresponding to 10% of a period of the stance phase S1 may be set as the threshold, or a predetermined fixed value (for example, 10 ms) may be set as the threshold. Furthermore, in the acceleration rate in the vertical axis direction, that is, in the vertical direction, a time of a peak Pb detected immediately after the start of the stance phase S1 may be set as a threshold, and a peak of the acceleration rate in the horizontal axis direction, that is, in the horizontal direction detected before that time may be specified as noise.

### [Processing related to display of result screen]

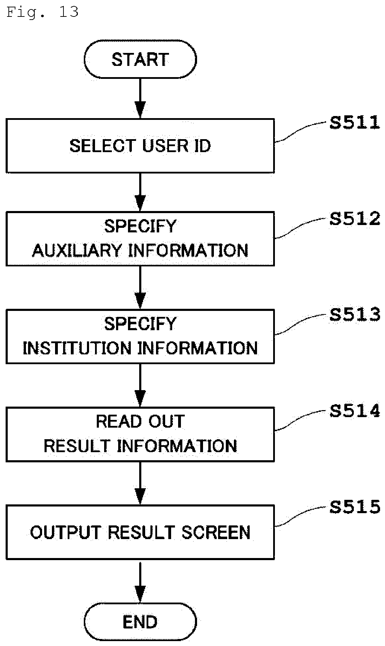

Fig. 13 illustrates a processing flow executed in the processing device 100 according to the embodiment of the present disclosure. Specifically, Fig. 13 illustrates a processing flow to be executed by the processor 111 for outputting a result screen displayed when it is determined in S113 in Fig. 8 that the measurement mode is not selected, or after various types of information subjected to the estimation processing in S316 in Fig. 10 is stored in the memory 112.

According to Fig. 13, first, the processor 111 receives the operation input by the operator through the operation input interface 113, and selects user ID information associated with a user whose information is desired to be displayed (S511). Then, the processor 111 refers to the user table and reads out the prognosis information and the level information associated with the selected user ID information. On the basis of the prognosis information and the level information which have been read out, the processor 111 refers to the auxiliary information tables corresponding thereto. At this time, the processor 111 may specify, as the auxiliary information, all pieces of the information stored in the auxiliary information table which have been referred to, or may specify only a part of the information by filtering (S512). For example, the processor 111 can narrow down the information stored in the auxiliary information table which has been referred to, on the basis of various pieces of information such as the gender, the height, the weight, and the age of the user stored, in advance, in the user table in association with the user ID information.

Similarly, on the basis of the prognosis information and the level information which have been read out, the processor 111 refers to the institution tables corresponding thereto. At this time, the processor 111 may specify, as the institution information, all pieces of the information stored in the institution table which have been referred to, or may specify only a part of the information by filtering (S513). For example, on the basis of the address information of the user stored, in advance, in the user table in association with the user ID information, and the position information stored in the institution table, the processor 111 can narrow down the institutions to an institution corresponding to the institution ID information which exists within a predetermined distance from the address, or 10 institutions in the order of proximity to the address.

Next, the processor 111 reads out information about the acceleration rate table associated with the user ID information, in addition to the peak width information, the peak number information, the appearance time information, the KAM value information, the prognosis information, and the level information associated with the user ID information which has been selected from the user table in S511, and controls the display 114 to output the result screen (S515).

### 5. Example of Result Screen

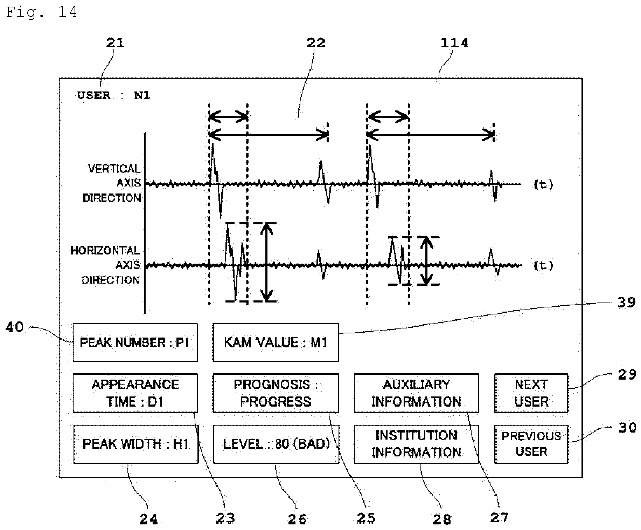

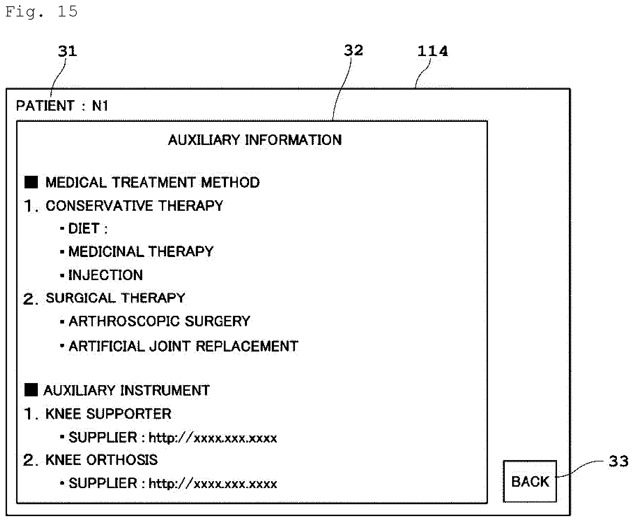

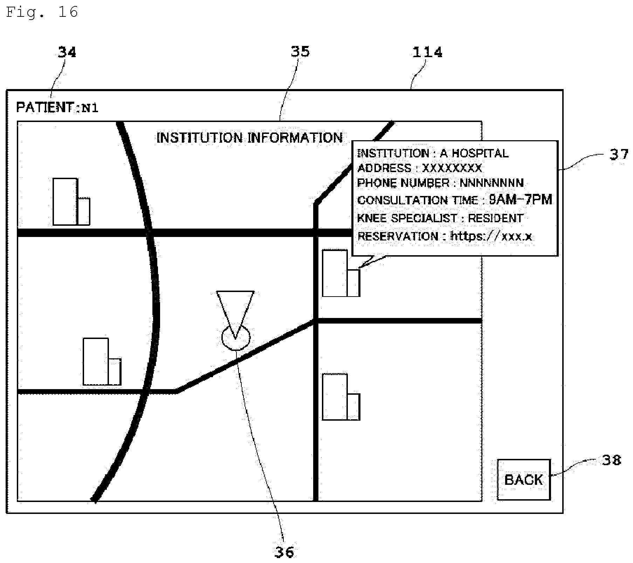

Fig. 14, 15, and 16 each illustrate an example of a screen displayed in the processing device 100 according to the embodiment of the present disclosure. Specifically, Fig. 14 is a diagram illustrating an example of a result screen first displayed when the result screen is outputted in S515 in Fig. 13. In addition, Fig. 15 and 16 are diagrams each illustrating an example of a result screen transitioned from the result screen in Fig. 14 when an instruction for displaying each piece of information is received on the result screen.

According to Fig. 14, the user information corresponding to the user ID information selected in S511 in Fig. 13 is displayed in a user information display area 21. Besides, in an output value display area 22 therebelow, the output value (acceleration rate) detected by the detection device 200 is displayed as a curve showing time in the X-axis direction and an output value in the Y-axis direction. Further below the area, there are an appearance time information display region 23, a peak width information display region 24, a prognosis information display region 25, a level information display region 26, a KAM value information display region 39, and a peak number information display region 40, and the information which has been read out in S515 in Fig. 13 is displayed in the corresponding regions. That is, by allowing doctors to browse information displayed in the output value display area 22 and the respective display areas 23-26, 39, and 40, it is possible to assist the doctors in estimating the current level of the condition of the knee (one example thereof is knee osteoarthritis) and the prognosis of knee osteoarthritis. In addition, next to these areas, there are an auxiliary information display icon 27 and an institution information display icon 28, and when an operation input on each icon from an operator is received, the screen shifts to a result screen on which respective pieces of information are displayed. Furthermore, next to these areas, a "next user icon 29" and a "previous user icon 30" for shifting to a result screen of a user associated with the preceding or subsequent user ID information are displayed in the order of users stored in the user table.

Fig. 15 illustrates one example of a result screen transitioned when the operation input from the operator on the auxiliary information display icon 27 in Fig. 14 is received through the operation input interface 113. According to Fig. 15, similarly to the result screen in Fig. 14, the user information corresponding to the user ID information selected in S511 in Fig. 13 is displayed in a user information display area 31. Further below the area, there is an auxiliary information display area 32, and the auxiliary information specified in S512 in Fig. 13 is displayed in this area. In the example in Fig. 15, information regarding medical treatment methods such as a conservative therapy and a surgical therapy, which are information useful for diagnosis and medical treatment by doctors who are operators, information about a supplier for auxiliary instruments, and the like are displayed from the information stored in the auxiliary information table. Furthermore, a "back icon 33" is displayed adjacent to the auxiliary information display area 32, and when an operation input on the icon is received, the screen returns to the result screen illustrated in Fig. 14.

Fig. 16 illustrates one example of a result screen transitioned when the operation input from the operator on the institution information display icon 28 in Fig. 14 is received through the operation input interface 113. According to Fig. 16, similarly to the result screen in Fig. 14, the user information corresponding to the user ID information selected in S511 in Fig. 13 is displayed in the user information display area 34. Further below the area, there is an institution information display area 35, and the institution information specified in S513 in Fig. 13 is displayed in this area. In the example in Fig. 15, respective institutions which exist within a predetermined distance on the basis of the address information of the user are displayed, together with the icons, from the information stored in the institution table. Specifically, a current location icon 36 indicates a position specified by the address information of the user, and on a map centered on that position, the icons of respective institutions are displayed on the basis of the specified position information of the respective institutions. Then, when an operation input on any one of these icons is received from the operator, a detailed information display area 37 pops up and is displayed in a superimposed manner. Specifically, the institution table is referred to on the basis of institution ID information about an institution corresponding to an icon for which the operation input has been received, and various pieces of information (institution name, address, telephone number, consultation hour information (time information), presence or absence of a specialist, reservation address) stored in the institution table is displayed. Furthermore, the "back icon 33" is displayed adjacent to the institution information display area 35, and when an operation input on the icon is received, the screen returns to the result screen illustrated in Fig. 14.

### 6. Example of Case of Using Trained Estimation Model

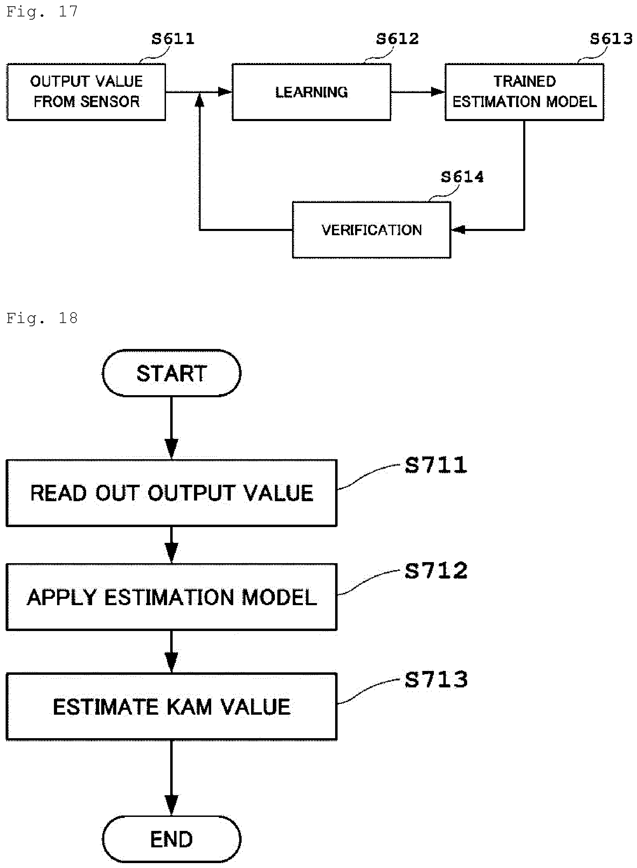

In the estimation processing in Fig. 11, a case of using the KAM conversion table in Fig. 7C has been described. However, it is also possible to use a trained KAM value estimation model in combination therewith or in place thereof. Fig. 17 illustrates a processing flow related to the generation of a trained estimation model according to the embodiment of the present disclosure. Specifically, Fig. 17 illustrates processing for generating a trained estimation model for estimating a KAM value used for estimating the prognosis of knee osteoarthritis in S416 in Fig. 11. This processing flow may be executed by the processor 111 of the processing device 100 or may be executed by a processor of another processing device.

According to Fig. 17, a step for acquiring an output value from the detection device 200 is executed (S611). As the output value, an output value detected by the detection device 200 mounted below the knee of the user suffering from knee osteoarthritis is used. Note that, as the output value, the acceleration rate in the horizontal direction detected at a predetermined cycle during a predetermined period can be used, or another output value can be used. Examples of the other output values include output values of six axes in total by further using the acceleration rate in the vertical direction, the acceleration rate in the depth direction, the angular velocity with respect to an axis in the horizontal direction, the angular velocity with respect to an axis in the vertical direction, and the angular velocity with respect to an axis in the depth direction, in addition to the acceleration rate in the horizontal direction. Then, a predetermined number of such output values are acquired for the purpose of deep learning.

Next, the output value acquired in S611 and a KAM value measured in advance as a correct answer label by another method are inputted as training data to a convolution neural network (CNN) for generating an estimation model, and learning is executed in a learning device including the convolution neural network so as to output the KAM value (S612). Then, a trained estimation model for estimating the KAM value is finally generated by repeating the learning in S612 (S613). Note that the KAM value as the correct answer label is measured by, for example, a method using motion capture.

Here, when the output value is detected in the detection device 200 for the trained estimation model which has been obtained, the KAM value may be separately calculated using another method, and verification may be performed using the output value and the KAM value (S614). Then, it is possible to adjust a parameter value used for the convolution neural network in response to the feedback for the result.

Note that, in the present disclosure, either a KAM peak value or a KAM area value can be used as the KAM value used for the output or the verification as described above. In the above discussion, the learning method using the convolution neural network has been explained as one example, but the present invention is not limited thereto. It is also possible to use other deep learning methods, or other machine learning methods. For example, it is also possible to prepare plural combinations of an output value from the detection device 200 and a KAM value for which a correspondence relation between the output value and the KAM value has been confirmed in advance, and generate a trained estimation model by using these combinations as teacher data.

Fig. 18 illustrates a processing flow executed in the processing device 100 according to the embodiment of the present disclosure. Specifically, Fig. 18 illustrates a specific processing flow, until the KAM value is estimated, of the estimation processing performed in S315 in Fig. 10.

According to Fig. 18, the processor 111 first reads out an output value associated with the user ID information which has been inputted or selected on the measurement screen from the acceleration rate table stored in the memory 112 (S711). Note that, although only the acceleration rate information is stored in Fig. 7A, the output values of six axes including angular velocity information may be stored as described above. Therefore, not only the acceleration rate information, but also the output values of six axes including angular velocity information can be read out as the output value read out in S711. Next, the processor 111 applies the read-out output value to the trained estimation model for estimating the KAM value generated in Fig. 17 (S712). Then, the processor 111 estimates the KAM value using the estimation model (S713). The processor 111 stores the estimated KAM value as the KAM value information in the user table in association with the user ID information. Note that processing similar to S416 in Fig. 11 is executed after the KAM value is estimated and stored.

As discussed above, in the present embodiment, it is possible to provide a processing device, program, method, and processing system which can be more simply used by a user when estimating a condition during exercise or assisting the estimation. Specifically, by using the output value detected by the detection device 200, it is possible to more simply estimate the current condition of the knee, for example, the condition of knee osteoarthritis, or assist the estimation, and estimate the prognosis of knee osteoarthritis or assist the estimation.

### 7. Other Embodiments

In the above embodiment, the case where the acceleration rate during exercise is detected using an acceleration sensor as the detection device 200 has been explained. However, in place of or in combination with the acceleration sensor, any sensor capable of detecting the exercise of the user 10, in particular, movements including bending and stretching of the knee, such as a gyro sensor, a geomagnetic sensor, and an expansion/contraction sensor can be used.

### 8. Example Illustrating Correlation between Output Value and Kam Value (example of KAM value estimation method in Fig. 11)

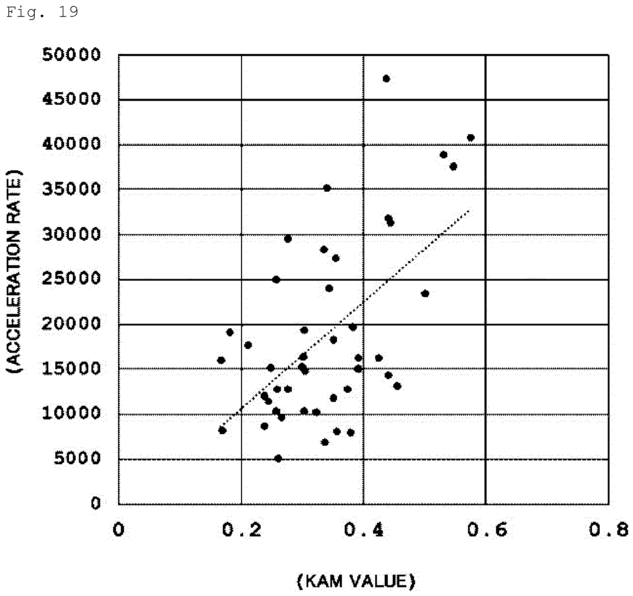

Fig. 19 illustrates a correlation between an acceleration rate at the beginning of the stance phase which has been actually measured, and a KAM value. Specifically, Fig. 19 is a diagram illustrating a relation between the peak width at the beginning of the stance phase calculated on the basis of the acceleration rate in the horizontal direction and the KAM value (KAM peak value) measured by motion capture, among a total of 44 output values which have been actually measured by the detection devices 200 attached below the knees of both legs of 22 subjects suffering from knee osteoarthritis. The KAM value was measured by use of a motion capture system "Oqus" (using eight 200 fps cameras) manufactured by Qualisys and a floor reaction force meter "AM 6110" (2000 Hz) manufactured by Bertec Corporation to detect the movement of reflective markers placed on each subject's standard 46 bone indices. Then, a KAM value curve was obtained by calculating the kinematic behavior of the knee by use of "Visual 3D" manufactured by C-motion, Inc. from the obtained movement of the reflective markers, thereby calculating a KAM peak value. On the other hand, as for the acceleration rate, small wireless multifunction sensors "TSND 151" manufactured by ATR-Promotions were attached below the knees of both legs of each subject to obtain an output value (acceleration rate) during walking, and a stance phase was specified from the obtained output value and an initial peak width was calculated. A Pearson correlation coefficient (p) was calculated on the basis of the obtained values, and when p<0.05, it was evaluated that there was a correlation therebetween. According to Fig. 19, it is shown that, in the measurement, the correlation coefficient between these numerical values was p<0.001, and it was confirmed that there is a high correlation between the KAM value and the peak width of the acceleration rate. That is, it was confirmed that the KAM value can be estimated by detecting the peak width of the acceleration rate at the beginning of the stance phase in the detection device 200.

### 9. Example 1 Illustrating Correlation between Output Value and KAM Value (example of KAM value estimation method in Fig. 18)

In addition, a case will be described in which output values of six axes in total, that is, the acceleration rate in the horizontal direction, the acceleration rate in the vertical direction, the acceleration rate in the depth direction, the angular velocity with respect to an axis in the horizontal direction, the angular velocity with respect to an axis in the vertical direction, and the angular velocity with respect to an axis in the depth direction, are used among a total of 44 output values which have been actually measured by the detection devices 200 attached below the knees of both legs of 22 subjects suffering from knee osteoarthritis, who are the same subjects as those in the example in Fig. 17. First, a trained estimation model was generated by using output values of 18 subjects randomly extracted from output values of 22 subjects, and a KAM area value or a KAM peak value prepared as a correct answer label. Note that, the KAM area value or the KAM peak value as the correct answer label was calculated from a KAM value curve obtained by a method, similar to that described above, with motion capture from the same 18 subjects. Next, in order to verify the generated estimation model, output values of the remaining four subjects were applied to the generated estimation model to estimate the KAM value. Then, the KAM value estimated by the estimation model was verified by being compared with the KAM area value or the KAM peak value calculated from the KAM value curve obtained by the same method with motion capture as that described above.

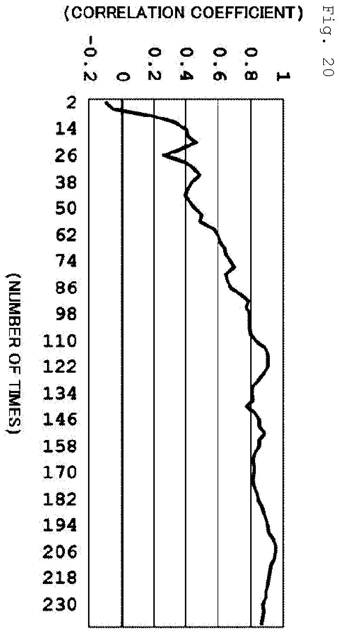

Fig. 20 is a diagram illustrating a correlation between a KAM area value estimated by applying the output values of the four subjects to the generated estimation model and a KAM area value calculated with motion capture. Specifically, Fig. 20 illustrates a graph in which the number of repetitions of learning in the estimation model (0 to 239 times) is assigned to the horizontal axis, and a correlation coefficient between a KAM area value obtained by applying the estimation model and a KAM area value obtained using motion capture is assigned to the vertical axis. According to Fig. 20, a correlation coefficient of 0.4 or more which is generally evaluated as "correlated" was stably obtained in the 47th and subsequent steps. In particular, an extremely high correlation coefficient of 0.934 at maximum was obtained during 239th learning steps. This indicates that the KAM area value estimated using the estimation model can be sufficiently used for evaluation of knee osteoarthritis and the like, similarly to the KAM area value obtained by motion capture.

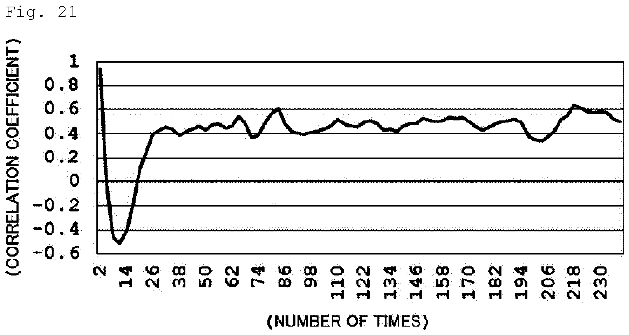

Fig. 21 is a diagram illustrating a correlation between a KAM peak value estimated by applying the output values of the four subjects to the generated estimation model and a KAM peak value calculated with motion capture. Specifically, Fig. 21 illustrates a graph in which the number of repetitions of learning in the estimation model (0 to 239 times) is assigned to the horizontal axis, and a correlation coefficient between a KAM peak value obtained by applying the estimation model and a KAM peak value obtained using motion capture is assigned to the vertical axis. According to Fig. 21, a correlation coefficient of 0.4 or more which is generally evaluated as "correlated" was stably obtained in the 29th and subsequent steps. In particular, an extremely high correlation coefficient of 0.633 at maximum was obtained during 218th learning steps. This indicates that the KAM peak value estimated using the estimation model can be sufficiently used for evaluation of knee osteoarthritis and the like, similarly to the KAM peak value obtained by motion capture.

### 10. Example 2 Illustrating Correlation between Output Value and KAM Value (example of KAM value estimation method in Fig. 18)

Newly, a case will be described in which output values of six axes in total, that is, the acceleration rate in the horizontal direction, the acceleration rate in the vertical direction, the acceleration rate in the depth direction, the angular velocity with respect to an axis in the horizontal direction, the angular velocity with respect to an axis in the vertical direction, and the angular velocity with respect to an axis in the depth direction, are used among a total of 61 output values which have been actually measured by the detection devices 200 attached below the knees of both legs of 31 subjects suffering from knee osteoarthritis. First, a trained estimation model was generated by using (49) output values of 25 subjects randomly extracted from output values of 31 subjects, and a KAM area value or a KAM peak value prepared as a correct answer label. Note that, the KAM area value or the KAM peak value as the correct answer label was calculated from a KAM value curve obtained by a method, similar to that described above, with motion capture from the same 25 subjects (49 output values). Next, in order to verify the generated estimation model, (12) output values of the remaining six subjects were applied to the generated estimation model to estimate the KAM value. Then, the KAM value estimated by the estimation model was verified by being compared with the KAM area value or the KAM peak value calculated from the KAM value curve obtained by the same method with motion capture as that described above.

First, a correlation between a KAM area value estimated by applying the (12) output values of the six subjects to the generated estimation model and a KAM area value calculated with motion capture was confirmed. Specifically, learning was repeatedly performed in the estimation model, and a correlation coefficient between a KAM area value obtained by applying the estimation model and a KAM area value obtained using motion capture was calculated. As a result, a correlation coefficient of 0.4 or more which is generally evaluated as "correlated" was stably obtained in the 200th and subsequent steps. In particular, an extremely high correlation coefficient of 0.8062 at maximum was obtained in the 2604th learning step. This indicates that the KAM area value estimated using the estimation model can be sufficiently used for evaluation of knee osteoarthritis and the like, similarly to the KAM area value obtained by motion capture.

Next, a correlation between a KAM peak value estimated by applying the (12) output values of the six subjects to the generated estimation model and a KAM peak value calculated with motion capture was confirmed. Specifically, learning was repeatedly performed in the estimation model, and a correlation coefficient between a KAM peak value obtained by applying the estimation model and a KAM peak value obtained using motion capture was calculated. As a result, a correlation coefficient of 0.4 or more was stably obtained in the 600th and subsequent steps. In particular, an extremely high correlation coefficient of 0.8603 at maximum was obtained in the 1129th learning step. This indicates that the KAM peak value estimated using the estimation model can be sufficiently used for evaluation of knee osteoarthritis and the like, similarly to the KAM peak value obtained by motion capture.

It is also possible to configure a system by appropriately combining or replacing respective components described in each embodiment.

The processing and procedures explained in the present specification can be realized not only by those explicitly described in the embodiments but also by software, hardware, or a combination thereof. Specifically, the processing and procedures explained in the present specification are realized by mounting logic corresponding to the processing on a medium such as an integrated circuit, a volatile memory, a nonvolatile memory, a magnetic disk, or an optical storage. In addition, the processing and procedures explained in the present specification can be implemented as a computer program to be executed by various computers including a processing device and a server device.

Even if it has been indicated that the processing and procedures explained in the present specification are executed by a single device, software, component, or module, such processing or procedures can be executed by a plurality of devices, a plurality of software products, a plurality of components, and/or a plurality of modules. Besides, even if it has been indicated that various types of information explained in the present specification are stored in a single memory or storage unit, such information can be stored in a distributed manner in a plurality of memories provided in a single device or a plurality of memories arranged distributedly in a plurality of devices. Furthermore, the software and hardware elements explained in the present specification can be realized by integrating them into fewer components or decomposing them into more components.

### Reference Signs List

- 1: processing system
- 100: processing device
- 200: detection device
- 300: server device
- 400: auxiliary tool

## Claims

1. A processing device comprising:
an input/output interface configured to receive an acceleration rate detected, in a wired or wireless manner, from a sensor which is attached to or around a knee of a leg of a human and is for detecting at least an acceleration rate of the human during exercise;
a memory configured to store computer readable instructions and the received acceleration rate, in addition to a predetermined instruction command; and
a processor configured to execute the computer readable instructions, by executing the predetermined instruction command stored in the memory, so as to:
estimating a condition of a knee joint of the human during exercise based on the acceleration rate.

2. The processing device according to claim 1, wherein the condition of the knee joint is estimated based on the acceleration rate after landing of the leg.

3. The processing device according to claim 1 or 2, wherein the condition of the knee joint is estimated based on a peak value of the acceleration rate detected after landing of the leg.

4. The processing device according to any one of claims 1 to 3, wherein the condition of the knee joint is estimated based on a number of peaks of the acceleration rate detected after landing of the leg.

5. The processing device according to any one of claims 1 to 4, wherein the condition of the knee joint is estimated based on the peak value of the acceleration rate detected after landing of the leg, and time taken from landing of the leg until the peak value is detected.

6. The processing device according to any one of claims 2 to 5, wherein the acceleration rate after landing of the leg is specified by detecting exercise of the leg in a vertical direction by using the sensor.

7. The processing device according to claim 6, wherein
the sensor detects both an acceleration rate in a horizontal direction and an acceleration rate in the vertical direction, and
the exercise of the leg in the vertical direction is detected based on the acceleration rate in the vertical direction.

8. The processing device according to any one of claims 1 to 7, wherein
the sensor detects both an acceleration rate in a horizontal direction and an acceleration rate in a vertical direction, and
the condition of the knee joint is estimated based on the acceleration rate in the horizontal direction.

9. The processing device according to claim 1, wherein the estimation of the condition of the knee joint is performed by a trained estimation model obtained through learning using the acceleration rate and information regarding the condition of the knee joint prepared in advance as a correct answer label.

10. The processing device according to any one of claims 1 to 9, wherein the condition of the knee joint is a symptom or a prognostic condition of a disease regarding the knee.

11. The processing device according to claim 10, wherein the processor is configured to output relevant information related to the prognostic condition in accordance with the prognostic condition that has been estimated.

12. The processing device according to claim 11, wherein the relevant information is updated at a predetermined timing.

13. A processing device comprising:
an input/output interface configured to receive an acceleration rate detected, in a wired or wireless manner, from a sensor which is attached to or around a knee of a leg of a human and is for detecting at least an acceleration rate of the human during exercise;
a memory configured to store computer readable instructions and the received acceleration rate, in addition to a predetermined instruction command; and
a processor configured to execute the computer readable instructions, by executing the predetermined instruction command stored in the memory, so as to:
outputting an acceleration rate after landing of the leg among the acceleration rates received from the sensor.

14. The processing device according to claim 13, wherein the acceleration rate after landing of the leg is specified by detecting exercise of the leg in a vertical direction by using the sensor.

15. The processing device according to claim 14, wherein
the sensor detects both an acceleration rate in a horizontal direction and an acceleration rate in the vertical direction, and
the exercise of the leg in the vertical direction is detected based on the acceleration rate in the vertical direction.

16. The processing device according to any one of claims 13 to 15, wherein
the sensor detects both the acceleration rate in the horizontal direction and the acceleration rate in the vertical direction, and
the acceleration rate in the horizontal direction is used for estimating a condition of a knee joint of the knee.

17. The processing device according to claim 13, wherein
a trained estimation model obtained through learning using the acceleration rate after landing of a leg and information regarding a condition of a knee joint of a knee prepared in advance as a correct answer label, is generated, and
with the estimation model, the condition of the knee joint of the knee is estimated using the acceleration rate outputted from the processor.

18. The processing device according to any one of claims 13 to 17, wherein
the processor is configured to output relevant information related to the knee of the human in accordance with the acceleration rate after landing of the leg.

19. The processing device according to claim 18, wherein the relevant information is updated at a predetermined timing.

20. A processing device comprising:
an input/output interface configured to receive an acceleration rate detected, in a wired or wireless manner, from a sensor which is attached to or around a knee of a leg of a human and is for detecting at least an acceleration rate of the human during exercise;
a memory configured to store computer readable instructions and the received acceleration rate, in addition to a predetermined instruction command; and
a processor configured to execute the computer readable instructions, by executing the predetermined instruction command stored in the memory, so as to:
estimating a degree or a prognosis of knee osteoarthritis based on the acceleration rate received from the sensor.

21. The processing device according to claim 20, wherein the degree or the prognosis of knee osteoarthritis is estimated by predicting an external knee adduction moment based on the acceleration rate.

22. The processing device according to claim 20 or 21, wherein the degree or the prognosis of knee osteoarthritis is estimated based on the acceleration rate after landing of the leg.

23. The processing device according to any one of claims 20 to 22, wherein the degree or the prognosis of knee osteoarthritis is estimated based on a peak value of the acceleration rate detected after landing of the leg.

24. The processing device according to any one of claims 20 to 23, wherein the degree or the prognosis of knee osteoarthritis is estimated based on a number of peaks of the acceleration rate detected after landing of the leg.

25. The processing device according to any one of claims 20 to 24, wherein the degree or the prognosis of knee osteoarthritis is estimated based on the peak value of the acceleration rate detected after landing of the leg, and time taken from landing of the leg until the peak value is detected.

26. The processing device according to any one of claims 20 to 25, wherein the acceleration rate after landing is specified by detecting exercise of the leg in a vertical direction by using the sensor.

27. The processing device according to claim 26, wherein
the sensor detects both an acceleration rate in a horizontal direction and an acceleration rate in the vertical direction, and
the exercise of the leg in the vertical direction is detected based on the acceleration rate in the vertical direction.

28. The processing device according to any one of claims 20 to 27, wherein
the sensor detects both the acceleration rate in the horizontal direction and the acceleration rate in the vertical direction, and
the degree of knee osteoarthritis is estimated based on the acceleration rate in the horizontal direction.

29. The processing device according to claim 20 or 21, wherein the estimation of the degree or the prognosis of knee osteoarthritis is performed by a trained estimation model obtained through learning using the acceleration rate and information regarding knee osteoarthritis prepared in advance as a correct answer label.

30. The processing device according to any one of claims 20 to 29, wherein
the processor is configured to output the relevant information about knee osteoarthritis in accordance with the degree of knee osteoarthritis.

31. The processing device according to claim 30, wherein the relevant information is updated at a predetermined timing.

32. A processing device comprising:
an input/output interface configured to receive an acceleration rate detected, in a wired or wireless manner, from a sensor which is attached to or around a knee of a leg of a human and is for detecting at least an acceleration rate of the human during exercise;
a memory configured to store computer readable instructions and the received acceleration rate, in addition to a predetermined instruction command; and
a processor configured to execute the computer readable instructions, by executing the predetermined instruction command stored in the memory, so as to:
outputting information indicating a condition of a knee joint of the human during exercise estimated based on the acceleration rate.

33. The processing device according to claim 32, wherein the information indicating the condition of the knee joint is information indicating a degree or a prognosis of knee osteoarthritis.

34. The processing device according to claim 32, wherein the information indicating the condition of the knee joint is information based on an external knee adduction moment of the knee joint.

35. A computer program product embodying computer readable instructions stored on a non-transitory computer-readable storage medium for causing a computer to execute a process by a processor, the computer comprising an input/output interface configured to receive an acceleration rate detected, in a wired or wireless manner, from a sensor which is attached to or around a knee of a leg of a human and is for detecting at least an acceleration rate of the human during exercise, and a memory configured to store the received acceleration rate, the computer configured to perform the steps of:
performing processing for estimating a condition of a knee joint of the human during exercise based on the acceleration rate.

36. A computer program product embodying computer readable instructions stored on a non-transitory computer-readable storage medium for causing a computer to execute a process by a processor, the computer comprising an input/output interface configured to receive an acceleration rate detected, in a wired or wireless manner, from a sensor which is attached to or around a knee of a leg of a human and is for detecting at least an acceleration rate of the human during exercise, and a memory configured to store the received acceleration rate, the computer configured to perform the steps of:
performing processing for outputting an acceleration rate after landing of the leg among the acceleration rates received from the sensor.

37. A computer program product embodying computer readable instructions stored on a non-transitory computer-readable storage medium for causing a computer to execute a process by a processor, the computer comprising an input/output interface configured to receive an acceleration rate detected, in a wired or wireless manner, from a sensor which is attached to or around a knee of a leg of a human and is for detecting at least an acceleration rate of the human during exercise, and a memory configured to store the received acceleration rate, the computer configured to perform the steps of:
performing processing for estimating a degree or a prognosis of knee osteoarthritis based on the acceleration rate received from the sensor.

38. A computer program product embodying computer readable instructions stored on a non-transitory computer-readable storage medium for causing a computer to execute a process by a processor, the computer comprising an input/output interface configured to receive an acceleration rate detected, in a wired or wireless manner, from a sensor which is attached to or around a knee of a leg of a human and is for detecting at least an acceleration rate of the human during exercise, and a memory configured to store the received acceleration rate, the computer configured to perform the steps of:
performing processing for outputting information indicating a condition of a knee joint of the human during exercise estimated based on the acceleration rate.

39. A method for causing a processor in a computer to execute a process, the computer comprising an input/output interface configured to receive an acceleration rate detected, in a wired or wireless manner, from a sensor which is attached to or around a knee of a leg of a human and is for detecting at least an acceleration rate of the human during exercise, and a memory configured to store computer readable instructions and the received acceleration rate, the processor executing the predetermined instruction command,
the method comprising a step for estimating a condition of a knee joint of the human during exercise based on the acceleration rate.

40. A method for causing a processor, in a computer to execute a process, the computer comprising an input/output interface configured to receive an acceleration rate detected, in a wired or wireless manner, from a sensor which is attached to or around a knee of a leg of a human and is for detecting at least an acceleration rate of the human during exercise, and a memory configured to store computer readable instructions and the received acceleration rate, the processor executing the predetermined instruction command,
the method comprising a step for outputting an acceleration rate after landing of the leg among the acceleration rates received from the sensor.

41. A method for causing a processor, in a computer to execute a process, the computer comprising an input/output interface configured to receive an acceleration rate detected, in a wired or wireless manner, from a sensor which is attached to or around a knee of a leg of a human and is for detecting at least an acceleration rate of the human during exercise, and a memory configured to store computer readable instructions and the received acceleration rate, the processor executing the predetermined instruction command,
the method comprising a step for estimating a degree or a prognosis of knee osteoarthritis based on the acceleration rate received from the sensor.

42. A method for causing a processor, in a computer to execute a process, the computer comprising an input/output interface configured to receive an acceleration rate detected, in a wired or wireless manner, from a sensor which is attached to or around a knee of a leg of a human and is for detecting at least an acceleration rate of the human during exercise, and a memory configured to store computer readable instructions and the received acceleration rate, the processor executing the predetermined instruction command,
the method comprising a step for outputting information indicating a condition of a knee joint of the human during exercise estimated based on the acceleration rate.

43. A processing system comprising:
the processing device according to any one of claims 1 to 34; and
a detection device including a sensor which is attached to or around a knee of a leg of a human and is for detecting at least an acceleration rate of the human during exercise.
